# EUROPEAN PATENT SPECIFICATION

(11) **EP 2 460 538 B1**
(45) Date of publication and mention of the grant of the patent: **13.09.2017**
(21) Application number: 10804532.9
(22) Date of filing: 30.07.2010
(51) Int. Cl.: C07K 16/28, A61K 39/395, A61P 35/04

(54) **CANCER METASTASIS INHIBITOR**
TUMORMETASTASENHEMMER
INHIBITEUR DE MÉTASTASES CANCÉREUSES

(30) Priority: 31.07.2009 JP 2009179780
(43) Date of publication of application: 06.06.2012
(73) Proprietor: Maeda, Shin, Tokyo 112-0001 (JP)
(72) Inventor: Maeda, Shin, Tokyo 112-0001 (JP)
(74) Representative: Vossius & Partner Patentanwälte Rechtsanwälte mbB
(86) International application number: PCT/JP2010/062874
(87) International publication number: WO 2011/013786

(56) References cited:
- EP-A1- 1 967 209
- WO-A1-2005/037315
- WO-A1-2007/061029
- WO-A1-2007/076927
- WO-A2-2006/119115
- JP-A- 6 237 772
- JP-T- 2005 524 606
- JP-T- 2008 538 931
- MAEDA SHIN ET AL: "Role of IKK beta/NF-kappa B activation for development of liver metastasis", HEPATOLOGY, vol. 46, no. 4, Suppl. S, October 2007 (2007-10), page 518A, XP002688348, & 58TH ANNUAL MEETING OF THE AMERICAN-ASSOCIATION-FOR-THE-STUDY-OF-LIVE R-DISEASES; BOSTON, MA, USA; NOVEMBER 02 -06, 2007 ISSN: 0270-9139
- MAEDA SHIN ET AL: "Essential roles of Ikk beta/Nf-kappa B activation for development of liver metastasis in mice", GASTROENTEROLOGY, vol. 130, no. 4, Suppl. 2, April 2006 (2006-04), page A750, XP002688349, & DIGESTIVE DISEASE WEEK MEETING/107TH ANNUAL MEETING OF THE AMERICAN-GASTROENTEROLOGICAL-ASSOCIATION; LOS ANGELES, CA, USA; MAY 19 -24, 2006 ISSN: 0016-5085
- SHIN MAEDA ET AL: "Ikappa B kinase[beta]/nuclear factor-[kappa]B activation controls the development of liver metastasis by way of interleukin-6 expression", HEPATOLOGY, vol. 50, no. 6, 1 December 2009 (2009-12-01), pages 1851-1860, XP055046049, ISSN: 0270-9139, DOI: 10.1002/hep.23199
- TAKEDA, K. ET AL.: 'Murine Tumor Cells Metastasizing Selectively in the Liver: Ability to Produce Hepatocyte-activating Cytokines Interleukin-1 and/or -6' JAPANEASE JOURNAL OF CANCER RESEARCH vol. 82, no. 11, 30 November 1991, pages 1299 - 1308, XP008152267
- YAMAKAWA, Y. ET AL.: 'Astrocytes Promote the Proliferation of Lung Cancer Cells in Brain Metastases via inflammatory cytokines, especially IL-6' NEUROSCIENCE vol. 48, no. 2/3, 01 June 2009, page 216, XP008153473

## Description

The present disclosure relates to cancer metastasis inhibitors comprising an IL-6 inhibitor as an active ingredient. Furthermore, the present disclosure relates to methods for suppressing cancer metastasis using cancer metastasis inhibitors comprising an IL-6 inhibitor as an active ingredient.

IL-6 is a cytokine also called B-cell stimulating factor 2 (BSF2) or interferon β2. IL-6 was discovered as a differentiation factor involved in the activation of B-cell lymphocytes (Non-Patent Document 1), and was later revealed to be a multifunctional cytokine that influences the function of various cells (Non-Patent Document 2). It has been reported to induce maturation of T lymphocytes (Non-Patent Document 3).

IL-6 transmits its biological activity via two kinds of proteins on the cell. The first is the IL-6 receptor, which is a ligand-binding protein to which IL-6 binds, with a molecular weight of about 80 kDa (Non-Patent Documents 4 and 5). The IL-6 receptor is present in a membrane-bound form that penetrates the cell membrane. It is expressed on the cell membrane, and also as a soluble IL-6 receptor, which mainly consists of the extracellular region of the membrane-bound form.

The other protein is the membrane protein gp130, which has a molecular weight of about 130 kDa and is involved in non-ligand binding signal transduction. The biological activity of IL-6 is transmitted into the cell through formation of an IL-6/IL-6 receptor complex by IL-6 and the IL-6 receptor followed by binding of gp130 to the complex (Non-Patent Document 6).

Metastasis (spreading and proliferation of cancer cells in a secondary organ) is the number one cause of cancer death (Non-Patent Document 7). For example, the liver is a site to which melanoma, colon cancer, and breast cancer frequently metastasize. When liver metastasis occurs, the natural course of the disease is associated with poor prognosis. Therefore, development of new therapeutic methods for metastatic liver cancer is in urgent need. Tumor metastasis progresses in a series of biological steps which move tumor cells from the primary organ to a distant site. During this process, tumor metastasis is controlled by various genetic and epigenetic factors (Non-Patent Document 8).

The nuclear factor (NF)-κB transcription factor is an important regulator of genes involved in inflammation and suppression of apoptosis (Non-Patent Document 9). In resting cells, NF-κ_{B} is maintained in the cytoplasm in an inactive state by binding to IκB. IκB is quickly degraded in response to stimuli such as tumor necrosis factor (TNF)-α and bacterial lipopolysaccharides (LPS), and this leads to the activation and nuclear entry of NF-κB (Non-Patent Document 10). This process requires IκB phosphorylation by the IκB kinase (IKK) complex which is composed of the three subunits, IKKα, IKKβ, and IKKγ. IKKβ is important for IκB degradation, and NF-κB activation in response to pathogen-associated molecular patterns (PAMPs) and proinflammatory stimuli.

The NF-κB transcription factor may function as an important factor that associates carcinogenesis with inflammation (Non-Patent Document 11). NF-κB was shown to be a factor for promoting tumorigenesis in colitis-associated cancer (CAC) (Non-Patent Document 12) and inflammation-associated cancer (Non-Patent Document 13). However, NF-κB activation may also function as an important regulator in cancers that are not associated with clear inflammation (Non-Patent Document 14). Matrix metalloproteinase and the serine protease urokinase-type plasminogen activation factor (uPA) play an important role in tumor infiltration and metastasis, and are regulated by NF-κB (Non-Patent Document 15). Cyclooxygenase (COX)-2, which is strongly regulated by NF-κB in a similar manner, is an inducible enzyme produced in many cell types in response to various stimuli. Recently, COX-2 overexpression was detected in several types of human cancers including colon cancer, breast cancer, prostate cancer, and pancreatic cancer, and COX-2 was shown to control various cellular processes including metastasis (Non-Patent Document 16). Furthermore, many NF-κB regulatory genes have been reported to be involved in tumor metastasis. Thus, NF-κB inhibition may provide alternative approaches for treatment of metastatic tumorigenesis.

IL-6 is a multifunctional cytokine which regulates immune and inflammatory responses, cell proliferation, and cell survival. However, since IL-6 has both tumor-promoting and tumor-suppressing functions, their functional relationship in tumorigenesis is still unclear. Recently, there have been several persuasive reports that describe mechanisms controlling IL-6 production in tumorigenesis (Non-Patent Documents 17 to 19). For example, Naugler *et al.* reported that estrogen inhibited IL-6 secretion in mice exposed to a chemical carcinogen (Non-Patent Document 17). The inhibition by estrogen may be the cause of male-specific increase in the onset of liver cancer observed in the same study. Furthermore, several studies reported that serum IL-6 levels are high in patients with various cancers, and this is associated with poor prognosis (Non-Patent Documents 20 and 21).

The prior-art documents related to the present disclosure are shown below.

### [Prior-art Documents]

### [Non-Patent Documents]

[Non-Patent Document 1] Hirano, T. et al., Nature (1986) 324, 73-76
[Non-Patent Document 2] Akira, S. et al., Adv. in Immunology (1993) 54, 1-78
[Non-Patent Document 3] Lotz, M. et al., J. Exp. Med. (1988) 167, 1253-1258
[Non-Patent Document 4] Taga, T. et al., J. Exp. Med. (1987) 166, 967-981
[Non-Patent Document 5] Yamasaki, K. etal., Science (1988) 241, 825-828
[Non-Patent Document 6] Taga, T. et al., Cell (1989) 58, 573-581
[Non-Patent Document 7] Steeg, P.S. Nat. Med. 12, 895-904 (2006)
[Non-Patent Document 8] Steeg, P.S. & Theodorescu, D. Nat Clin Pract Oncol 5, 206-219 (2008)
[Non-Patent Document 9] Karin, M. & Lin, A. Nat Immunol 3, 221-227 (2002)
[Non-Patent Document 10] Ghosh, S. & Karin, M. Cell 109 Suppl, S81-96 (2002)
[Non-Patent Document 11] Karin, M., et al., Nat Rev Cancer 2, 301-310 (2002)
[Non-Patent Document 12] Greten, F.R., et al. Cell 118, 285-296 (2004)
[Non-Patent Document 13] Pikarsky, E., et al. Nature 431, 461-466 (2004)
[Non-Patent Document 14] Maeda, S., et al. Cell 121, 977-990 (2005)
[Non-Patent Document 15] Bond, M., et al. FEBS Lett. 435, 29-34 (1998)
[Non-Patent Document 16] Sarkar, F.H., et al. Mini Rev Med Chem 7, 599-608 (2007)
[Non-Patent Document 17] Naugler, W.E., et al. Science 317, 121-124 (2007)
[Non-Patent Document 18] Gao, S.P., et al. J. Clin. Invest. 117, 3846-3856 (2007)
[Non-Patent Document 19] Sansone, P., et al. J. Clin. Invest. 117, 3988-4002 (2007)
[Non-Patent Document 20] Ashizawa, T., et al. Gastric Cancer 8, 124-131 (2005)
[Non-Patent Document 21] Matzaraki, V., et al. Clin. Biochem. 40, 336-342 (2007)

### _[Problems to be Solved]

Accordingly, an objective of the present invention is to provide novel inhibitors of cancer metastasis.

### [Means for Solving the Problems]

The present inventors used a model of liver metastasis induced through the spleen to determine whether NF-κB activation in the liver is associated with the onset of metastatic tumors. Liver cell-specific deletion of IKKβ which prevents NF-κB activation in liver cells did not affect the onset of metastatic tumors. In contrast, when IKKβ was deleted from both liver cells and hematopoietically-derived cells, the onset of tumors was decreased remarkably. Tumor cells activated neighboring hematopoietic cells (Kupffer cells) and produced mitogens such as interleukin (IL)-6, and this promoted angiogenesis and tumor growth. The mitogen production depended on NF-κB in hematopoietic Kupffer cells. Furthermore, treatment with an anti-IL-6 receptor antibody reduced the degree of metastatic tumor development, and this indicates that IL-6 is involved in liver metastasis.

That is, the present inventors showed that tumor metastasis depends on inflammation, and proinflammatory intervention that targets Kupffer cells is useful for chemical prevention of metastatic tumors.

Thus, the present invention relates to an agent for use in a method of inhibiting cancer metastasis comprising an IL-6 inhibitor as an active ingredient, wherein the IL-6 inhibitor is an anti-IL-6 receptor antibody. The present invention also relates to the use of an IL-6 inhibitor for the manufacture of a medicament for inhibiting cancer metastasis, wherein the IL-6 inhibitor is an anti-IL-6 receptor antibody. Said cancer metastasis may be cancer metastasis to the liver, e.g. cancer metastasis of lung cancer to the liver. According to the invention said anti-IL-6 receptor antibody may be a chimeric, humanized, or human antibody.

Also provided herein is the following:
[1] a cancer metastasis inhibitor comprising an interleukin-6 (IL-6) inhibitor as an active ingredient;
[2] the metastasis inhibitor of [1], which suppresses cancer metastasis to the liver;
[3] the metastasis inhibitor of [1] or [2], wherein the IL-6 inhibitor is an IL-6 receptor inhibitor;
[4] the metastasis inhibitor of [3], wherein the IL-6 receptor inhibitor is a human IL-6 receptor inhibitor;
[5] the metastasis inhibitor of [3] or [4], wherein the IL-6 receptor inhibitor is an anti-IL-6 receptor antibody;
[6] the metastasis inhibitor of [5], wherein the anti-IL-6 receptor antibody is a chimeric, humanized, or human antibody;
[7] the metastasis inhibitor of any one of [1] to [6], which suppresses metastasis of lung cancer to the liver;
[8] a method for suppressing cancer metastasis, which comprises the step of administering an IL-6 inhibitor to a subject;
[9] the method of [8], which suppresses cancer metastasis to the liver;
[10] the method of [8] or [9], wherein the IL-6 inhibitor is an IL-6 receptor inhibitor;
[11] the method of [10], wherein the IL-6 receptor inhibitor is a human IL-6 receptor inhibitor;
[12] the method of [10] or [11], wherein the IL-6 receptor inhibitor is an anti-IL-6 receptor antibody;
[13] the method of [12], wherein the anti-IL-6 receptor antibody is a chimeric, humanized, or human antibody;
[14] the method of any one of [8] to [13], which suppresses metastasis of lung cancer to the liver;
[15] use of an IL-6 inhibitor for production of a cancer metastasis inhibitor;
[16] the use of [15], which suppresses cancer metastasis to the liver;
[17] the use of [15] or [16], wherein the IL-6 inhibitor is an IL-6 receptor inhibitor;
[18] the use of [17], wherein the IL-6 receptor inhibitor is a human IL-6 receptor inhibitor;
[19] the use of [17] or [18], wherein the IL-6 receptor inhibitor is an anti-IL-6 receptor antibody;
[20] the use of [19], wherein the anti-IL-6 receptor antibody is a chimeric, humanized, or human antibody;
[21] the use of any one of [15] to [20], which suppresses metastasis of lung cancer to the liver;
[22] an IL-6 inhibitor for use in a method for suppressing cancer metastasis;
[23] the IL-6 inhibitor of [22], which suppresses cancer metastasis to the liver;
[24] the IL-6 inhibitor of [21] or [22], which is an IL-6 receptor inhibitor;
[25] the IL-6 inhibitor of [24], wherein the IL-6 receptor inhibitor is a human IL-6 receptor inhibitor;
[26] the IL-6 inhibitor of [24] or [25], wherein the IL-6 receptor inhibitor is an anti-IL-6 receptor antibody;
[27] the IL-6 inhibitor of [26], wherein the anti-IL-6 receptor antibody is a chimeric, humanized, or human antibody; and
[28] the IL-6 inhibitor of any one of [22] to [27], which suppresses metastasis of lung cancer to the liver.

### Brief Description of the Drawings

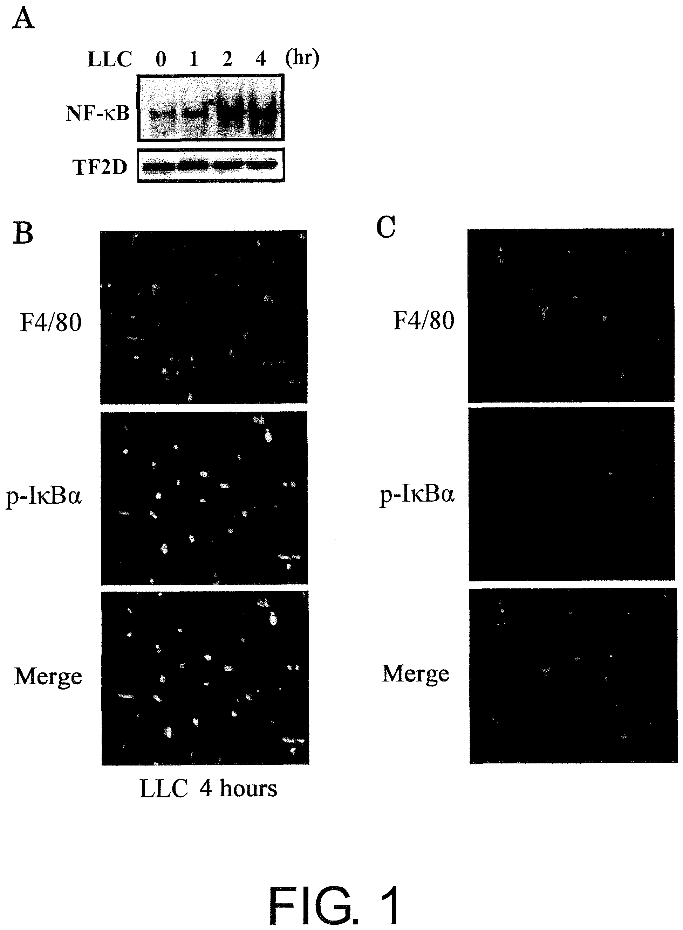

Fig. 1 presents photographs showing the relationship between NF-κB activation and liver metastasis of LLC cells. (A) NF-κB DNA binding activity in liver tissues collected from LLC-injected mice was determined using electrophoretic mobility shift assay (EMSA). The recovery of nuclear proteins was determined by immunoblotting with an anti-TF2D antibody. (B) Four hours after LLC injection, liver tissues were stained using anti-phospho-IκBα and anti-F4/80 antibodies. (C) Four hours after sham operation, liver tissues were stained with anti-phospho-IκBα and anti-F4/80 antibodies.

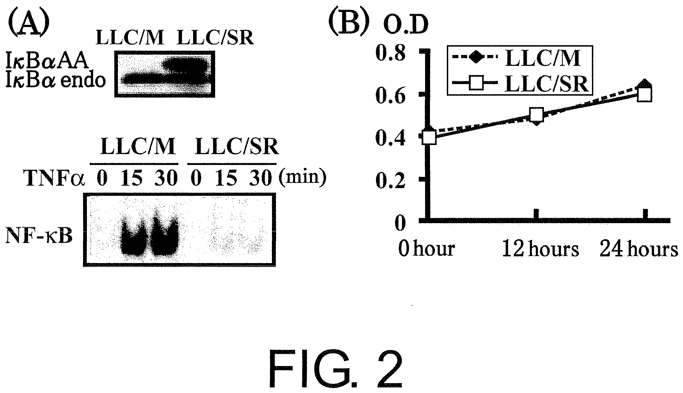

Fig. 2 presents a diagram and photographs showing the involvement of NF-κB activation in liver metastasis of LLC cells. (A) LLC cells were stably transfected with an empty vector (IκBα/M) or s-rIκB (LLC/SR), and the expression of endogenous IκBα and transfected s-rIκB was determined by immunoblotting with anti-IκBα (upper panel). NF-κB activation by TNFα in LLC/M and LLC/SR was analyzed by EMSA (lower panel). (B) Cell growth was determined using Cell Counting Kit-8 (Dojindo, Kumamoto, Japan). OD450/570 was determined at 0, 12, and 24 hours for each cell line.

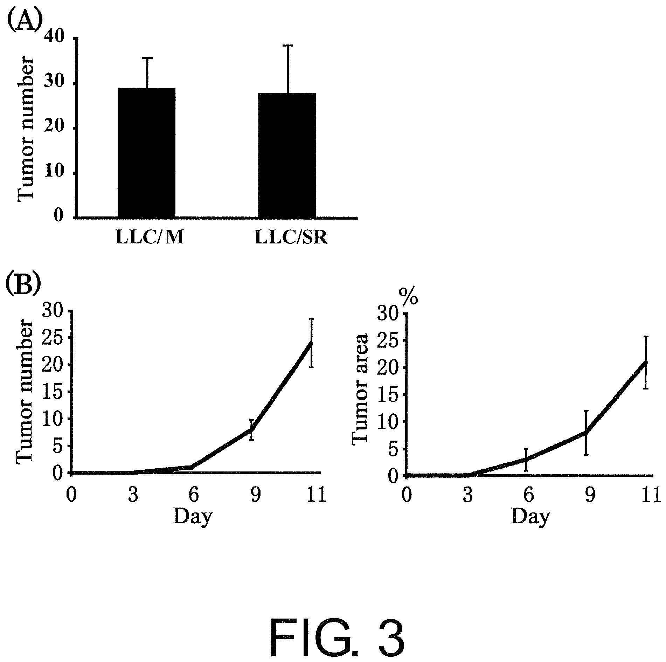

Fig. 3 presents diagrams showing the involvement of NF-κB activation in liver metastasis of LLC cells. (A) The number of metastatic tumors in each liver was counted on day 11 post-injection of LLC/M (n = 5) or LLC/SR (n = 5) cells. (B) The number of tumors (≥ 0.5 mm) and the tumor-occupied area (%) in the large hepatic lobe of LLC-injected WT mice.

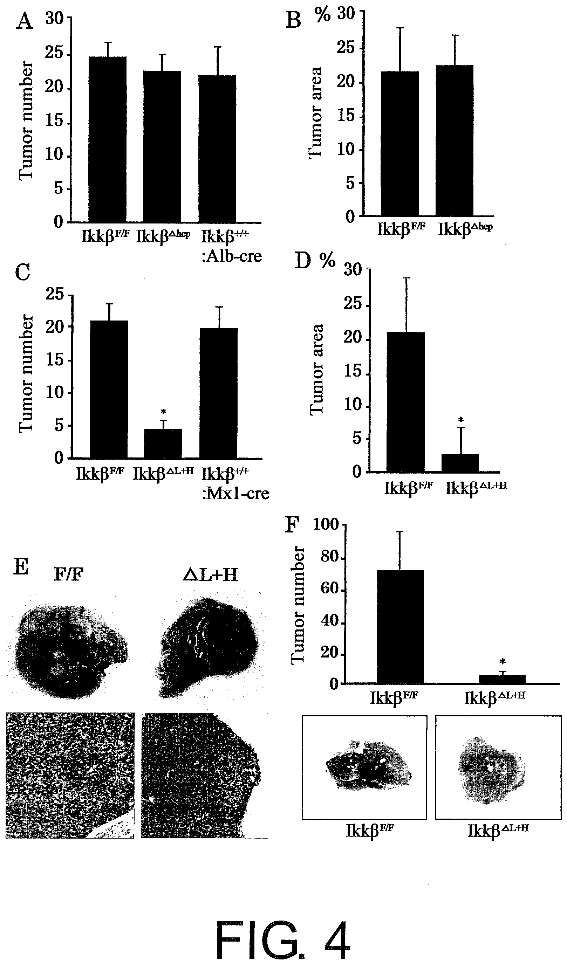

Fig. 4 presents diagrams and photographs showing low-level tumor metastasis in Ikkβ^{ΔL+H} mice. (A) The number of metastatic tumors in the liver collected from Ikkβ^{F/F} (n = 8), Ikkβ^{Δhep} (n = 9), or Ikkβ^{+/+}:Alb-cre (n = 6) mice was counted on day 11 post-LLC injection. (B) Fraction of the tumor-occupied area in the large hepatic lobe (the number of mice and the treatment are as mentioned above). (C) LLC cells were injected into poly (IC)-injected Ikkβ^{F/F}, Ikkβ^{F/F}:Mx1-Cre, or Ikkβ^{+/+}:Mx1-Cre mice. The number of metastatic tumors was counted in Ikkβ^{F/F} (n = 7), Ikkβ^{ΔL+H} (n = 10), or Ikkβ^{+/+}:Mx1-Cre (n = 6) mice on day 11 post-LLC injection. (D) Fraction of the tumor-occupied area in the large hepatic lobe (the number of mice and the treatment are as mentioned above). (E) Representative livers of Ikkβ^{F/F} and Ikkβ^{ΔL+H} mice on day 11 post-LLC injection (upper panel). Typical liver histology of Ikkβ^{F/F} and Ikkβ^{ΔL+H} mice (lower panel; 40 x magnification, hematoxylin-eosin staining). (F) The number of metastatic tumors in the liver of Ikkβ^{F/F} (n = 5) or Ikkβ^{L+H} (n = 4) mice was counted on day 11 post-injection of B16/F10 cells (upper panel). The liver of Ikkβ^{F/F} and Ikkβ^{ΔL+H} mice on day 11 post-B16/F10 injection (lower panel). The values are represented as mean ± standard error of the mean (SEM). * P < 0.05 as determined using Student's t-test.

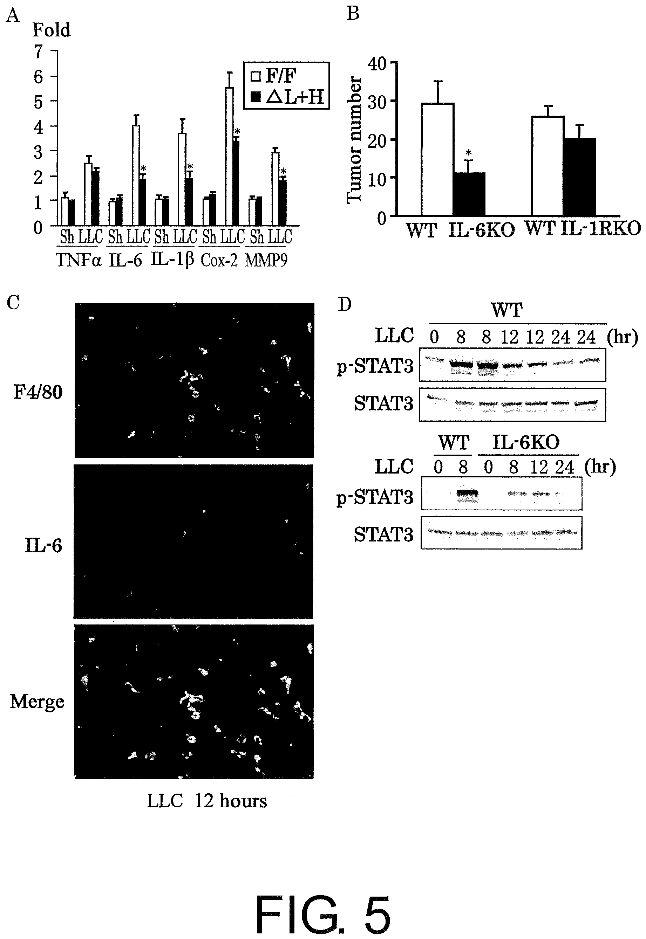

Fig. 5 presents diagrams and photographs showing a low-level expression of IL-6 in Ikkβ^{ΔL+H} mice and lower tumor metastasis in IL-6 knockout mice. (A) LLC or PBS (sh) was injected into mice of the indicated genotype, and the liver was removed four hours later. Total mRNA was isolated, and specific mRNA expression was quantified by real-time PCR. The values show the mean from three experiments. (B) The number of metastatic tumors in the liver of wild-type (WT) (n = 10 for each), IL-6 knockout (IL-6KO) (n = 12), and IL-1R knockout (IL-1RKO) (n = 10) mice was determined on day 11 post-LLC injection. The values are represented as the mean ± standard error of the mean (SEM). * P < 0.05 as determined using Student's t-test. (C) Frozen liver sections were prepared 12 hours after LLC injection, and immunostaining was performed for the expression of IL-6 and F4/80. (D) LLC cells were injected into the spleen of WT and IL-6 KO mice, and the mice were sacrificed at the indicated times. Cell lysates were prepared and the levels of phospho-STAT3 and STAT3 were determined by immunoblotting.

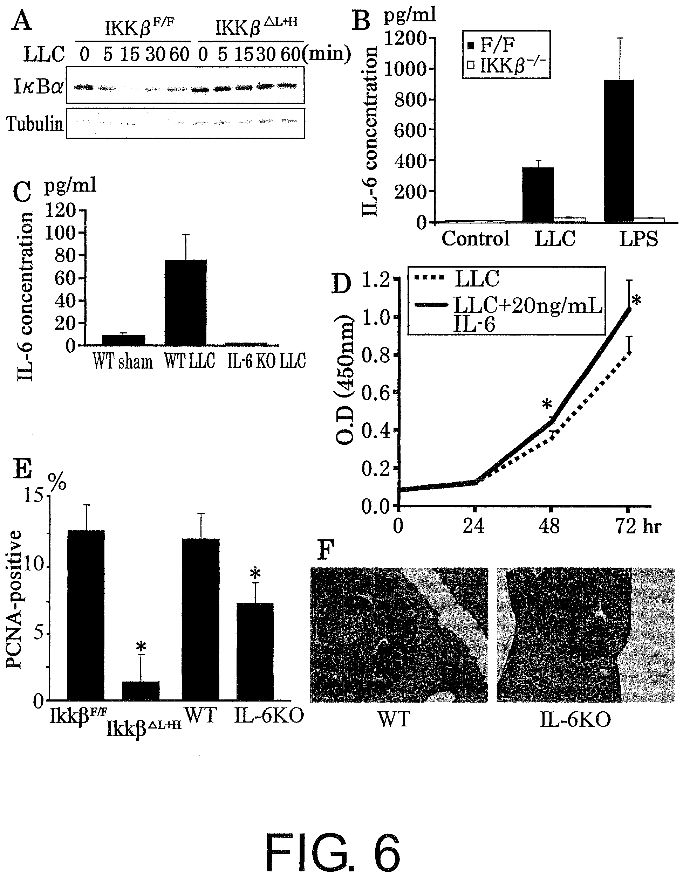

Fig. 6 presents diagrams and photographs showing VEGF expression-mediated enhancement of tumor angiogenesis by IL-6. (A) Bone marrow-derived macrophages (BMDM) isolated from Ikkβ^{F/F} and Ikkβ^{ΔL+H} mice were incubated with an LLC culture supernatant. Cell lysates were prepared at the indicated times, and the protein expression of IκBα and tubulin was determined by immunoblotting. (B) BMDM from Ikkβ^{F/F} and Ikkβ^{ΔL+H} mice was incubated for 24 hours with or without (control) an LLC culture supernatant or LPS (100 ng/mL). Then, the IL-6 levels were determined by ELISA. (C) Sera were collected from LLC-injected and sham-operated mice on day 11, and the IL-6 levels were determined by ELISA. (D) LLC cells were treated with or without IL-6 (20 ng/mL). Cell growth was determined using a cell counting kit. OD450/570 was measured at the indicated time points. * P < 0.05 as determined using Student's t-test. (E) A tissue of the largest lobe of an LLC-injected liver was immunohistochemically stained with an anti-PCNA antibody, and positive cells were estimated. * P < 0.05 as determined using Student's t-test. (F) Typical examples of tumor tissues derived from LLC-injected WT and IL-6KO, which were immunohistochemically stained with anti-PCNA (x 100 magnification).

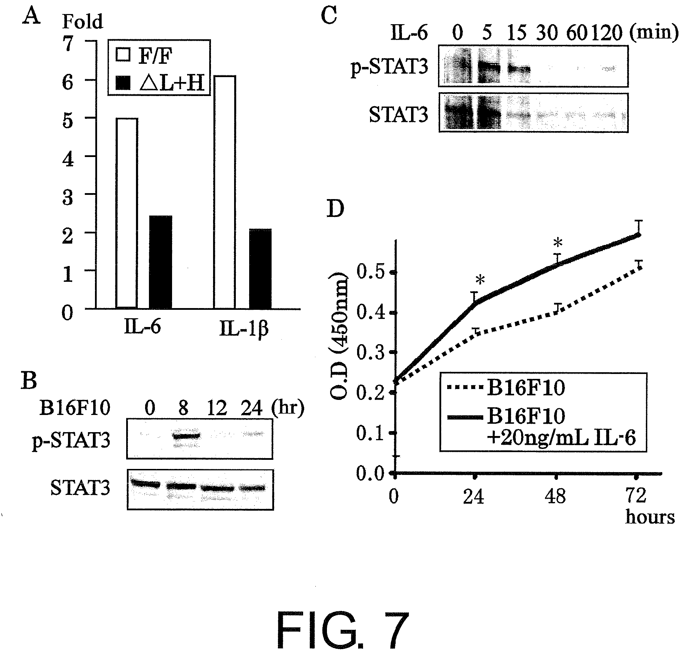

Fig. 7 presents diagrams and photographs showing the effect of IL-6 on B16F10 cells. (A) B16F10 was injected into mice of the indicated genotype and the liver was removed four hours later. Total mRNA was isolated, and specific mRNA expression was quantified by real-time PCR. (B) B16F10 cells were injected into the spleen of WT mice and they were sacrificed at the indicated times. Cell lysates were prepared and the levels of phospho-STAT3 and STAT3 were determined by immunoblotting. (C) B16F10 cells were treated with IL-6 (20 ng/mL). Cell lysates were prepared at the indicated times and the levels of phospho-STAT3 (p-STAT3) and total STAT3 were determined by immunoblotting. (D) B16F10 cells were treated with or without IL-6 (20 ng/mL). Cell growth was determined using a cell counting kit. OD450/570 was measured at the indicated time points. * P < 0.05 as determined using Student's t-test.

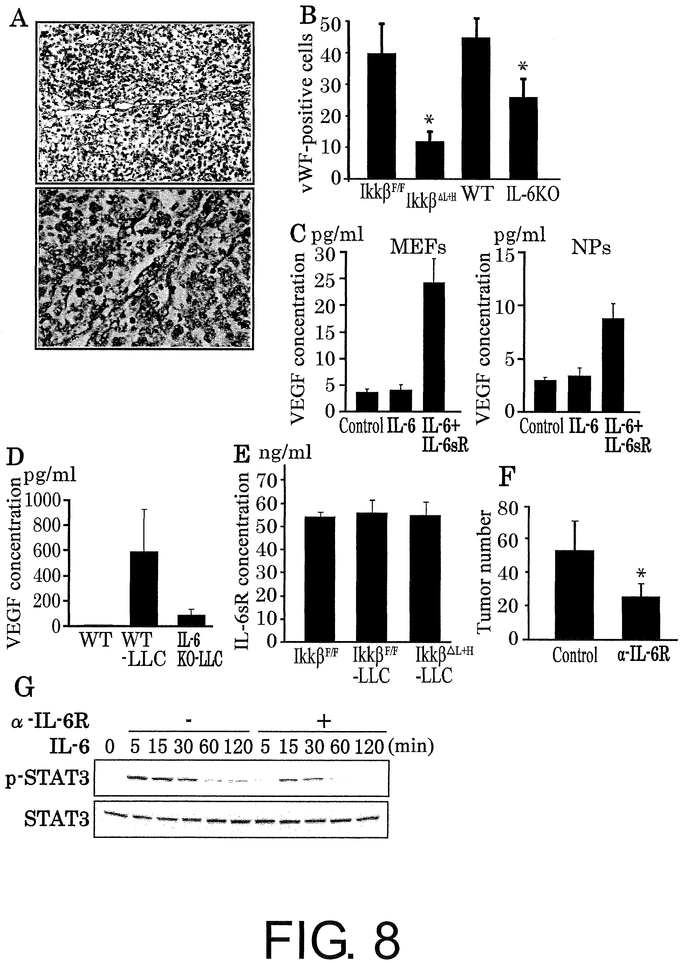

Fig. 8 presents diagrams and photographs showing that inhibition of IL-6 signals decreases liver metastasis. (A) Tumors were immunohistochemically stained using an anti-vWF antibody to estimate the vascularity of the tumors (magnification: 100 x in the upper panel, 400 x in the lower panel). (B) The number of vWF-positive cells per view was determined under a microscope (x 100 magnification; n = 5 for each genotype). (C) Liver non-parenchymal cells (NP) and mouse embryonic fibroblasts (MEF) prepared from wild-type (WT) mice were treated with IL-6 in the presence or absence of an IL-6 receptor. The VEGF levels were determined by ELISA. (D) Sera were collected from LLC-injected WT (n = 5) and IL-6KO (n = 5) mice, and non-injected mice on day 11, and the VEGF levels were determined by ELISA. (E) LLC was injected or not into mice having the indicated genotypes (n = 5 for each), and the IL-6sR levels were determined by ELISA on day 11. (F) WT mice were treated with a neutralizing anti-IL-6 antibody (n = 9) or control antibody (n = 9) on days 0, 3, 6, and 9 after LLC injection. The number of metastatic tumors in mice on day 11 post-LLC injection is indicated. The values are presented as the mean ± standard error of the mean (SEM). * P < 0.05 as determined using Student's t-test. (G) LLC cells were treated with IL-6 in the presence or absence of a neutralizing anti-IL-6 receptor antibody (α-IL-6R). Cell lysates were prepared at the indicated times and the levels of phospho-STAT3 (p-STAT3) and total STAT3 were determined by immunoblotting.

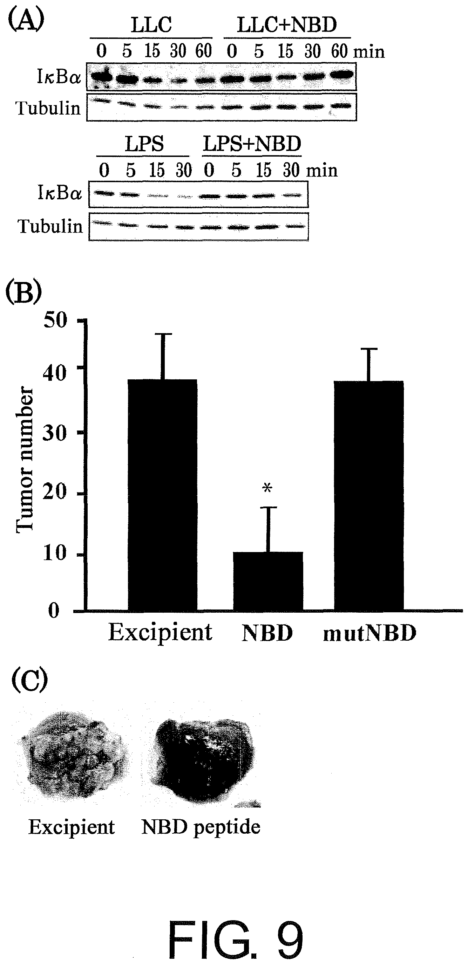

Fig. 9 presents a diagram and photographs showing inhibition of tumor metastasis by the NEMO-binding domain (NBD). (A) J774.1 cells were treated with an LLC cell culture supernatant or LPS (100 ng/mL) in the presence or absence of an NBD peptide. Cell lysates were prepared at the indicated times and the levels of IκBα and tubulin were determined by immunoblotting. (B) Wild-type (WT) mice were treated with an NBD peptide (n = 10), mutant (mut) NBD peptide (n = 4), or medium (PBS; n = 9) on days 0, 3, 6, and 9 after LLC injection. The number of metastatic tumors on day 11 post-LLC injection is shown. The values are represented as the mean ± standard error of the mean (SEM). * P < 0.05 as determined using Student's t-test. (C) The liver of a PBS-treated or NBD-treated mouse on day 11 post-LLC injection.

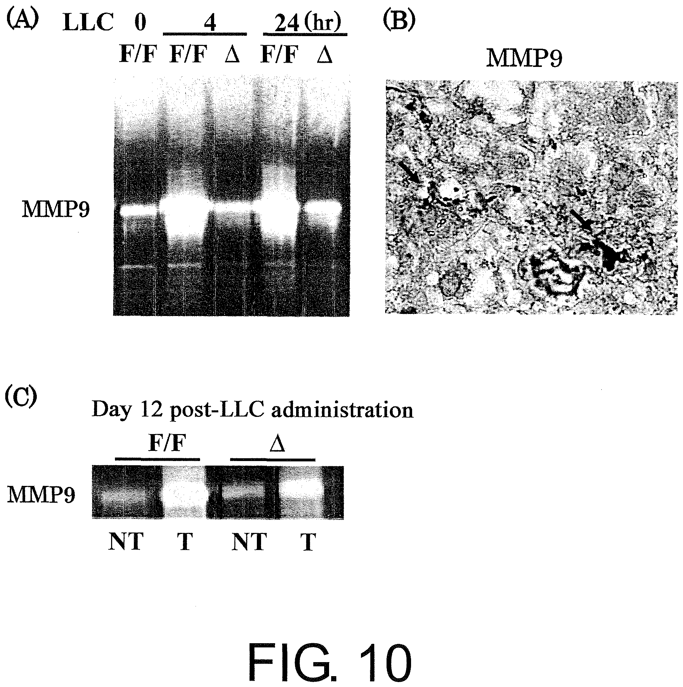

Fig. 10 presents photographs showing that MMP9 is activated in the liver of LLC-injected mice in an IKKβ-dependent manner. (A) LLC was injected into mice of the indicated genotype, and the liver was removed 4 hours or 24 hours later. Total proteins were extracted, and zymography was performed for MMP9. (B) Frozen liver sections were prepared 12 hours after LLC injection, and MMP9 expression was detected by immunostaining. (C) LLC was injected into mice of the indicated genotype, and the liver was removed 11 days later. Total proteins were extracted, and zymography was performed for MMP9.

### Mode for Carrying Out the Disclosure

In the present invention, "IL-6 inhibitor" refers to a substance that blocks IL-6-mediated signal transduction and inhibits the biological activity of IL-6. Specific examples of IL-6 inhibitors include substances that bind to IL-6, substances that inhibit IL-6 expression, substances that bind to an IL-6 receptor, substances that inhibit the expression of an IL-6 receptor, substances that bind to gp130, and substances that inhibit gp130 expression. Without particular limitation, IL-6 inhibitors include anti-IL-6 antibodies, anti-IL-6 receptor antibodies, anti-gp130 antibodies, IL-6 variants, soluble IL-6 receptor variants, partial peptides of IL-6, and partial peptides of an IL-6 receptor, as well as low-molecular-weight compounds, antisense, siRNAs and such that show an equivalent activity.

Preferably, IL-6 inhibitors include IL-6 receptor inhibitors.

In the present invention, "IL-6 receptor inhibitor" refers to a substance that blocks IL-6 receptor-mediated signal transduction and inhibits the biological activity of IL-6. An IL-6 receptor inhibitor is preferably a substance that binds to an IL-6 receptor, and has activity to inhibit the binding between IL-6 and an IL-6 receptor.

Examples of IL-6 receptor inhibitors include anti-IL-6 receptor antibodies, soluble IL-6 receptor variants, partial peptides of an IL-6 receptor, and low-molecular-weight substances that show an equivalent activity, but they are not particularly limited thereto. Preferred examples of the IL-6 receptor inhibitors include antibodies that recognize an IL-6 receptor.

There is no particular limitation on the origin of an anti-IL-6 receptor antibody used in the present invention; however, for example, the antibody is preferably derived from a mammal, and more preferably derived from human.

Anti-IL-6 receptor antibodies used in the present invention can be obtained as polyclonal or monoclonal antibodies using a known means. In particular, the anti-IL-6 receptor antibodies used in the present invention are preferably mammal-derived monoclonal antibodies. Mammal-derived monoclonal antibodies include those produced by hybridomas and those produced by a host transformed with an expression vector carrying an antibody gene using genetic engineering techniques. Anti-IL-6 receptor antibodies block transmission of the biological activity of IL-6 into cells by binding to an IL-6 receptor thereby inhibiting the binding of IL-6 to the IL-6 receptor. Examples of such antibodies include the MR16-1 antibody (Tamura, T. et al. Proc. Natl. Acad. Sci. USA (1993) 90, 11924-11928), PM-1 antibody (Hirata, Y. et al., J. Immunol. (1989) 143, 2900-2906), AUK12-20 antibody, AUK64-7 antibody, and AUK146-15 antibody (International Patent Publication WO 92-19759). Of them, the PM-1 antibody is an example of preferred monoclonal antibodies against the human IL-6 receptor, and the MR16-1 antibody is an example of preferred monoclonal antibodies against the mouse IL-6 receptor; however, the antibodies are not limited thereto.

Basically, anti-IL-6 receptor monoclonal antibody-producing hybridomas can be prepared using known techniques as follows. Specifically, immunization is carried out by a conventional immunization method using as a sensitizing antigen an IL-6 receptor. The resulting immune cells are fused with known parental cells by a conventional cell fusion method. Then, monoclonal antibody-producing cells are screened using a conventional screening method.

Specifically, anti-IL-6 receptor antibodies can be produced as follows. For example, a human IL-6 receptor or mouse IL-6 receptor for use as a sensitizing antigen for obtaining antibodies can be produced using the IL-6 receptor genes and/or amino acid sequences disclosed in European Patent Application Publication No. EP 325474 and Japanese Patent Application Kokai Publication No. (JP-A) Hei 3-155795, respectively.

There are two types of IL-6 receptor proteins: one expressed on the cell membrane and the other separated from the cell membrane (soluble IL-6 receptors) (Yasukawa, K. et al., J. Biochem. (1990) 108, 673-676). The soluble IL-6 receptor essentially consists of the extracellular region of the cell membrane-bound IL-6 receptor, and differs from the membrane-bound IL-6 receptor in that it lacks the transmembrane region or both the transmembrane and intracellular regions. Any IL-6 receptor may be employed as an IL-6 receptor protein, as long as it can be used as a sensitizing antigen for producing an anti-IL-6 receptor antibody used in the present invention.

After transforming an appropriate host cell with a known expression vector system into which an IL-6 receptor gene sequence has been inserted, the IL-6 receptor protein of interest is purified from the inside of the host cell or from the culture supernatant using a known method. This purified IL-6 receptor protein may be used as a sensitizing antigen. Alternatively, a cell expressing the IL-6 receptor or a fusion protein of the IL-6 receptor protein and another protein may be used as a sensitizing antigen.

Mammals to be immunized with a sensitizing antigen are not particularly limited, but are preferably selected considering compatibility with the parental cell used for cell fusion. Generally, rodents such as mice, rats, and hamsters are used.

Animals are immunized with sensitizing antigens according to known methods. For example, as a general method, animals are immunized by intraperitoneal or subcutaneous injection of a sensitizing antigen. Specifically, the sensitizing antigen is preferably diluted or suspended in an appropriate amount of phosphate-buffered saline (PBS), physiological saline or such, mixed with an appropriate amount of a general adjuvant (e.g., Freund's complete adjuvant), emulsified, and then administered to a mammal several times, every four to 21 days. In addition, an appropriate carrier may be used for immunization with a sensitizing antigen.

Following such immunization, an increased level of a desired antibody in serum is confirmed and then immune cells are obtained from the mammal for cell fusion. Preferred immune cells for cell fusion include, in particular, spleen cells.

The mammalian myeloma cells used as parental cells, *i. e.* as partner cells to be fused with the above immune cells, include various known cell strains, for example, P3X63Ag8.653 (Kearney, J. F. et al., J. Immunol (1979) 123, 1548-1550), P3X63Ag8U.1 (Current Topics in Microbiology and Immunology (1978) 81, 1-7), NS-1 (Kohler, G. and Milstein, C., Eur. J. Immunol. (1976) 6, 511-519), MPC-11 (Margulies, D. H. et al., Cell (1976) 8, 405-415), SP2/0 (Shulman, M. et al., Nature (1978) 276, 269-270), F0 (de St. Groth, S. F. et al., J. Immunol. Methods (1980) 35, 1-21), S194 (Trowbridge, I. S., J. Exp. Med. (1978) 148, 313-323), R210 (Galfre, G. et al., Nature (1979) 277, 131-133), and such.

Basically, cell fusion of the aforementioned immune cells and myeloma cells can be performed using known methods, for example, the method of Milstein *et al.* (Kohler, G. and Milstein, C., Methods Enzymol. (1981) 73, 3-46), and such.

More specifically, the aforementioned cell fusion is achieved in general nutrient culture medium in the presence of a cell fusion enhancing agent. For example, polyethylene glycol (PEG), Sendai virus (HVJ), and such are used as fusion enhancing agents. Further, to enhance fusion efficiency, auxiliary agents such as dimethyl sulfoxide may be added depending on the need.

The ratio of immune cells to myeloma cells used is preferably, for example, 1 to 10 immune cells for each myeloma cell. The culture medium used for the aforementioned cell fusion is, for example, the RPMI 1640 or MEM culture medium, which are suitable for proliferation of the aforementioned myeloma cells. A general culture medium used for culturing this type of cell can also be used. Furthermore, serum supplements such as fetal calf serum (FCS) can be used in combination.

For cell fusion, the fusion cells (hybridomas) of interest are formed by mixing predetermined amounts of an aforementioned immune cell and myeloma cell in an aforementioned culture medium, and then adding and mixing a concentration of 30% to 60% (w/v) PEG solution (e.g., a PEG solution with a mean molecular weight of about 1,000 to 6,000) pre-heated to about 37°C. Then, cell fusion agents and such that are unsuitable for the growth of hybridomas can be removed by repeatedly adding an appropriate culture medium and then removing the supernatant by centrifugation.

The above hybridomas are selected by culturing cells in a general selection culture medium, for example, HAT culture medium (a culture medium containing hypoxanthine, aminopterin, and thymidine). Culture in HAT culture medium is continued for a sufficient period, generally several days to several weeks, to kill cells other than the hybridomas of interest (unfused cells). Then, a standard limited dilution method is performed to screen and clone hybridomas that produce an antibody of interest.

In addition to the methods for immunizing non-human animals with antigens for obtaining the aforementioned hybridomas, desired human antibodies with the activity of binding to a desired antigen or antigen-expressing cell can be obtained by sensitizing a human lymphocyte with a desired antigen protein or antigen-expressing cell *in vitro,* and fusing the sensitized B lymphocyte with a human myeloma cell (*e.g.,* U266) (see, Japanese Patent Application Kokoku Publication No. (JP-B) Hei 1-59878 (examined, approved Japanese patent application published for opposition)). Further, a desired human antibody can be obtained by administering an antigen or antigen-expressing cell to a transgenic animal that has a repertoire of human antibody genes, and then following the aforementioned method (see, International Patent Application Publication Nos. WO 93/12227, WO 92/03918, WO 94/02602, WO 94/25585, WO 96/34096, and WO 96/33735).

The hybridomas thus prepared which produce monoclonal antibodies can be subcultured in a conventional culture medium and stored in liquid nitrogen for a long period.

When obtaining monoclonal antibodies from the aforementioned hybridomas, the following methods may be employed: a method in which the hybridomas are cultured according to a conventional method and the antibodies are obtained as a culture supernatant; a method in which the hybridomas are proliferated by administering them to a compatible mammal and the antibodies are obtained as ascites; etc. The former method is preferred for obtaining antibodies with high purity, and the latter is preferred for large-scale antibody production.

For example, anti-IL-6 receptor antibody-producing hybridomas can be prepared by the method disclosed in JP-A (Kokai) Hei 3-139293. Such hybridomas can be prepared by injecting a PM-1 antibody-producing hybridoma into the abdominal cavity of a BALB/c mouse, obtaining ascites, and then purifying a PM-1 antibody from the ascites; or by culturing the hybridoma in an appropriate medium (e.g., RPMI 1640 medium containing 10% fetal bovine serum, and 5% BM-Condimed H1 (Boehringer Mannheim); hybridoma SFM medium (GIBCO-BRL); PFHM-II medium (GIBCO-BRL), *etc*.) and then obtaining PM-1 antibody from the culture supernatant.

Herein, recombinant antibodies can be used as the monoclonal antibodies, wherein the antibodies are produced using genetic recombination techniques by cloning an antibody gene from a hybridoma, inserting the gene into an appropriate vector, and then introducing the vector into a host (see, for example, Borrebaeck, C. A. K. and Larrick, J. W., Therapeutic Monoclonal Antibodies, published in the United Kingdom by Macmillan Publishers Ltd, 1990).

More specifically, mRNAs encoding antibody variable (V) regions are isolated from cells that produce antibodies of interest, such as hybridomas. mRNAs can be isolated by preparing total RNAs according to known methods, such as the guanidine ultracentrifugation method (Chirgwin, J. M. et al., Biochemistry (1979) 18, 5294-5299) and the AGPC method (Chomczynski, P. et al., Anal. Biochem. (1987) 162, 156-159), and preparing mRNAs using the an mRNA Purification Kit (Pharmacia) and such. Alternatively, mRNAs can be directly prepared using a QuickPrep mRNA Purification Kit (Pharmacia).

cDNAs of the antibody V regions are synthesized from the obtained mRNAs using reverse transcriptase. cDNAs may be synthesized using an AMV Reverse Transcriptase First-strand cDNA Synthesis Kit and so on. Further, to synthesize and amplify the cDNAs, the 5'-RACE method (Frohman, M. A. et al., Proc. Natl. Acad. Sci. USA (1988) 85, 8998-9002; Belyavsky, A. et al., Nucleic Acids Res. (1989) 17, 2919-2932) using 5'-Ampli FINDER RACE Kit (Clontech) and PCR may be employed. A DNA fragment of interest is purified from the obtained PCR products and then ligated with a vector DNA. Then, a recombinant vector is prepared using the above DNA and introduced into *Escherichia coli* or such, and then its colonies are selected to prepare a desired recombinant vector. The nucleotide sequence of the DNA of interest is confirmed by, for example, the dideoxy method.

When a DNA encoding the V region of an antibody of interest is obtained, the DNA is ligated with a DNA that encodes a desired antibody constant region (C region), and inserted into an expression vector. Alternatively, a DNA encoding an antibody V region may be inserted into an expression vector comprising a DNA of an antibody C region.

To produce an antibody to be used in the present invention, as described below, an antibody gene is inserted into an expression vector such that it is expressed under the control of an expression regulating region, for example, an enhancer and promoter. Then, the antibody can be expressed by transforming a host cell with this expression vector.

To reduce heteroantigenicity against humans and such, artificially modified genetic recombinant antibodies such as chimeric antibodies and humanized antibodies can be used. These modified antibodies can be prepared using known methods.

A chimeric antibody can be obtained by ligating a DNA encoding an antibody V region obtained as mentioned above, with a DNA encoding a human antibody C region, then inserting this into an expression vector and introducing it into a host for production (see, European Patent Application Publication No. EP 125023; International Patent Application Publication No. WO 92/19759). This known method can be used to obtain chimeric antibodies useful for the present invention.

Humanized antibodies are also referred to as reshaped human antibodies, and are antibodies wherein the complementarity determining regions (CDRs) of an antibody from a mammal other than human (*e.g*., a mouse antibody) are transferred into the CDRs of human antibodies. General methods for this gene recombination are also known (see, European Patent Application Publication No. EP 125023, International Patent Application Publication No. WO 92/19759).

More specifically, DNA sequences designed such that the CDRs of a mouse antibody are ligated with the framework regions (FRs) of a human antibody are synthesized by PCR from several oligonucleotides produced to contain overlapping portions at their termini. The obtained DNA is ligated with a human antibody C region-encoding DNA and then inserted into an expression vector. The expression vector is introduced into a host to produce the humanized antibody (see, European Patent Application Publication No. EP 239400, International Patent Application Publication No. WO 92/19759).

The human antibody FRs to be ligated via the CDRs are selected so that the CDRs form suitable antigen binding sites. The amino acid(s) within the FRs of the antibody variable regions may be substituted as necessary so that the CDRs of the reshaped human antibody form an appropriate antigen binding site (Sato, K. et al., Cancer Res. (1993) 53, 851-856).

Human antibody C regions are used for the chimeric and humanized antibodies. Human antibody heavy chain C regions include Cγ, etc. For example, Cγ1, Cγ2, Cγ3, or Cγ4 may be used. Human antibody light chain C regions include, for example, Cκ and Cλ. Furthermore, to improve the stability of the antibodies or their production, the human antibody C regions may be modified.

Chimeric antibodies consist of the variable region of an antibody derived from a non-human mammal and the constant region of an antibody derived from a human; humanized antibodies consist of the CDRs of an antibody derived from a non-human mammal and the framework regions and constant regions derived from a human antibody. They have reduced antigenicity in the human body, and are thus useful as antibodies used for the present invention.

Preferred specific examples of humanized antibodies for use in the present invention include a humanized PM-1 antibody (see International Patent Publication No. WO 92-19759), and an antibody comprising an amino acid sequence having one or more amino acid sequence substitutions, deletions, additions, and/or insertions in the amino acid sequence of a humanized PM-1 antibody. A more specific example is tocilizumab. Other specific examples include the antibodies described in WO2009/041621.

Furthermore, in addition to the aforementioned methods for obtaining human antibodies, techniques for obtaining human antibodies by panning using a human antibody library are also known. For example, the variable regions of human antibodies can be expressed on phage surfaces as single chain antibodies (scFv) by using the phage display method, and antigen-binding phages can then be selected. By analyzing the genes of the selected phages, DNA sequences encoding the human antibody variable regions that bind to the antigen can be determined. Once the DNA sequence of an scFv that binds to the antigen is revealed, an appropriate expression vector comprising the sequence can be constructed to obtain a human antibody. These methods are already known, and the publications of WO 92/01047, WO 92/20791, WO93/06213, WO 93/11236, WO 93/19172, WO 95/01438, and WO 95/15388 can be used as reference.

The antibody genes constructed as mentioned above can be expressed according to conventional methods. When a mammalian cell is used, the antibody gene can be expressed using a DNA in which the antibody gene to be expressed is functionally ligated to a useful commonly used promoter and a poly A signal downstream of the antibody gene, or a vector comprising the DNA. Examples of a promoter/enhancer include the human cytomegalovirus immediate early promoter/enhancer.

Furthermore, other promoters/enhancers that can be used for expressing the antibodies for use in the present invention include viral promoters/enhancers from retroviruses, polyoma viruses, adenoviruses, simian virus 40 (SV40), and such; and also include mammalian cell-derived promoters/enhancers such as human elongation factor 1α (HEF1α).

For example, when the SV40 promoter/enhancer is used, the expression can be easily performed by following the method by Mulligan *et al.* (Mulligan, R. C. et al., Nature (1979) 277, 108-114). Alternatively, in the case of the HEF1α promoter/enhancer, the method by Mizushima *et al.* (Mizushima, S. and Nagata S., Nucleic Acids Res. (1990) 18, 5322) can be easily used.

Production systems using prokaryotic cells include those using bacterial cells. Known bacterial cells include *E. coli* and *Bacillus subtilis.*

When *E. coli* is used, an antibody gene can be expressed by functionally ligating a conventional promoter, a signal sequence for antibody secretion, and the antibody gene to be expressed. Examples of the promoter include a lacZ promoter, araB promoter and such. When a lacZ promoter is used, genes can be expressed according to the method of Ward *et al.* (Ward, E. S. et al., Nature (1989) 341, 544-546; Ward, E. S. et al., FASEB J. (1992) 6, 2422-2427); and the araB promoter may be used according to the method of Better *et al.* (Better, M. et al., Science (1988) 240, 1041-1043).

When the antibody is produced into the periplasm of *E. coli,* the pel B signal sequence (Lei, S. P. et al., J. Bacteriol. (1987) 169, 4379-4383) may be used as a signal sequence for antibody secretion. The antibodies produced into the periplasm are isolated, and then used after appropriately refolding the antibody structure (see, for example, WO 96/30394).

As the replication origin, those derived from SV40, polyoma virus, adenovirus, bovine papilloma virus (BPV) and such may be used. In addition, to enhance the gene copy number in a host cell system, the expression vector may comprise the aminoglycoside phosphotransferase (APH) gene, thymidine kinase (TK) gene, *E. coli* xanthine-guanine phosphoribosyltransferase (Ecogpt) gene, dihydrofolate reductase (dhfr) gene, or such as a selection marker.

Any production system may be used to prepare the antibodies for use in the present invention. The production systems for antibody preparation include *in vitro* and *in vivo* production systems. *In vitro* production systems include those using eukaryotic cells or prokaryotic cells.

When eukaryotic cells are used as hosts, the production systems include those using animal cells, plant cells, or fungal cells. Such animal cells include: (1) mammalian cells, for example, CHO, COS, myeloma, baby hamster kidney (BHK), HeLa, Vero, and such; (2) amphibian cells, for example, *Xenopus* oocyte; and (3) insect cells, for example, sf9, sf21, Tn5, and such. Known plant cells include cells derived from *Nicotiana tabacum,* which may be cultured as a callus. Known fungal cells include yeasts such as *Saccharomyces (e.g., S. cerevisiae*), mold fungi such as *Aspergillus* (*e.g.*, *A. niger*), and such.

Antibodies can be obtained by using transformation to introduce an antibody gene of interest into these cells, and then culturing the transformed cells *in vitro.* Cultures are conducted according to known methods. For example, DMEM, MEM, RPMI 1640, or IMDM may be used as the culture medium, and serum supplements such as FCS may be used in combination. Furthermore, cells into which antibody genes have been introduced may be transferred into the abdominal cavity or such of an animal to produce the antibodies *in vivo.*

On the other hand, *in vivo* production systems include those using animals or plants. Production systems using animals include those that use mammals or insects.

Mammals that can be used include goats, pigs, sheep, mice, bovines and such (Vicki Glaser, SPECTRUM Biotechnology Applications, 1993). Furthermore, insects that can be used include silkworms. When using plants, tobacco may be used, for example.

An antibody gene is introduced into these animals or plants, the antibody is produced in the body of the animals or plants, and this antibody is then recovered. For example, an antibody gene can be prepared as a fusion gene by inserting it into the middle of a gene encoding a protein such as goat β casein, which is uniquely produced into milk. DNA fragments comprising the fusion gene, which includes the antibody gene, are injected into goat embryos, and the embryos are introduced into female goats. The desired antibody is obtained from milk produced by the transgenic animals born to the goats that received the embryos, or produced from progenies of these animals. The transgenic goats can be given hormones to increase the volume of milk containing the desired antibody that they produce (Ebert, K. M. et al., Bio/Technology (1994) 12, 699-702).

When silkworms are used, the silkworms are infected with a baculovirus into which a desired antibody gene has been inserted, and the desired antibody is obtained from the body fluids of these silkworms (Maeda, S. et al., Nature (1985) 315, 592-594). Moreover, when tobacco is used, the desired antibody gene is inserted into a plant expression vector (e.g., pMON530) and the vector is introduced into bacteria such as *Agrobacterium tumefaciens.* This bacterium is used to infect tobacco (*e.g., Nicotiana tabacum*) such that desired antibodies can be obtained from the leaves of this tobacco (Julian, K.-C. Ma et al., Eur. J. Immunol. (1994) 24, 131-138).

When producing antibodies using *in vitro* or *in vivo* production systems as described above, DNAs encoding an antibody heavy chain (H chain) and light chain (L chain) may be inserted into separate expression vectors and a host is then co-transformed with the vectors. Alternatively, the DNAs may be inserted into a single expression vector for transforming a host (see International Patent Application Publication No. WO 94/11523).

The antibodies used in the present invention may be antibody fragments or modified products thereof, as long as they can be suitably used in the present invention. For example, antibody fragments include Fab, F(ab')2, Fv, and single chain Fv (scFv), in which the Fvs of the H and L chains are linked by an appropriate linker.

Specifically, the antibody fragments are produced by treating antibodies with enzymes, for example, papain or pepsin; or alternatively, genes encoding these fragments are constructed and introduced into expression vectors, and they are expressed in appropriate host cells (see, for example, Co, M. S. et al., J. Immunol. (1994) 152, 2968-2976; Better, M. & Horwitz, A. H., Methods in Enzymology (1989) 178, 476-496; Plueckthun, A. & Skerra, A., Methods in Enzymology (1989) 178, 497-515; Lamoyi, E., Methods in Enzymology (1989) 121, 652-663; Rousseaux, J. et al., Methods in Enzymology (1989) 121, 663-666; Bird, R. E. et al., TIBTECH (1991) 9, 132-137).

An scFv can be obtained by linking the H-chain V region and the L-chain V region of an antibody. In the scFv, the H-chain V region and the L-chain V region are linked by a linker, preferably a peptide linker (Huston, J. S. et al., Proc. Natl. Acad. Sci. USA (1988) 85, 5879-5883). The V regions of the H and L chains in an scFv may be derived from any of the antibodies described above. Peptide linkers for linking the V regions include, for example, arbitrary single chain peptides consisting of 12 to 19 amino acid residues.

An scFv-encoding DNA can be obtained by using a DNA encoding an H chain or a V region and a DNA encoding an L chain or a V region of the aforementioned antibodies as templates, and using PCR to amplify a DNA portion that encodes the desired amino acid sequence in the template sequence and uses primers that define the termini of the portion, and then further amplifying the amplified DNA portion with a DNA that encodes a peptide linker portion and primer pairs that link both ends of the linker to the H chain and L chain.

Once an scFv-encoding DNA has been obtained, an expression vector comprising the DNA and a host transformed with the vector can be obtained according to conventional methods. In addition, scFv can be obtained according to conventional methods using the host.

As mentioned above, these antibody fragments can be produced from the host by obtaining and expressing their genes. Herein, "antibodies" includes such antibody fragments.

Antibodies bound to various molecules, such as polyethylene glycol (PEG), may also be used as modified antibodies. Herein, "antibodies" includes such modified antibodies. These modified antibodies can be obtained by chemically modifying the obtained antibodies. Such methods have already been established in the art.

Antibodies produced and expressed as mentioned above can be isolated from the inside or outside of the cells or from the hosts, and then purified to homogeneity. The antibodies for use in the present invention can be isolated and/or purified using affinity chromatography. Columns to be used for the affinity chromatography include, for example, protein A columns and protein G columns. Carriers used for the protein A columns include, for example, HyperD, POROS, Sepharose FF and such. Alternatively, other methods used for the isolation and/or purification of common proteins may be used; however, there is no limitation on the methods.

For example, the antibodies used for the present invention may be isolated and/or purified by appropriately selecting and combining chromatography other than affinity chromatography, filters, ultrafiltration, salting-out, dialysis, and such. Chromatography includes, for example, ion-exchange chromatography, hydrophobic chromatography, gel filtration, and such. The Chromatography can be applied to high performance liquid chromatography (HPLC). Alternatively, reverse phase HPLC may be used.

The concentration of an antibody obtained as mentioned above can be determined by absorbance measurement, ELISA, or such. Specifically, absorbance is determined by appropriately diluting the antibody solution with PBS(-), and measuring absorbance at 280 nm, and calculating the concentration (1.35 OD = 1 mg/ml). Alternatively, when using ELISA, the measurement can be performed as follows. Specifically, 100 µl of goat anti-human IgG (TAG) diluted to 1 µg/ml with 0.1 M bicarbonate buffer (pH 9.6) is added to a 96-well plate (Nunc) and incubated overnight at 4°C to immobilize the antibody. After blocking, 100 µl of an appropriately diluted antibody or an appropriately diluted sample comprising the antibody, and human IgG (CAPPEL) are added as a standard, and incubated for one hour at room temperature.

After washing, 100 µl of 5,000x diluted alkaline phosphatase-labeled anti-human IgG (BIO SOURCE) is added and incubated for one hour at room temperature. After another wash, a substrate solution is added and incubated, and the absorbance at 405 nm is measured using Microplate Reader Model 3550 (Bio-Rad) to calculate the concentration of the antibody of interest.

The IL-6 receptor partial peptides are peptides that comprise part or all of the amino acid sequence of the region of the IL-6 receptor amino acid sequence that is involved in the binding between IL-6 and the IL-6 receptor. Such peptides usually comprise ten to 80, preferably 20 to 50, more preferably 20 to 40 amino acid residues.

The IL-6 receptor partial peptides can be produced according to generally known methods, for example, genetic engineering techniques or peptide synthesis methods, by specifying the region of the IL-6 receptor amino acid sequence that is involved in the binding between IL-6 and the IL-6 receptor, and using a portion or entirety of the amino acid sequence of the specified region.

When preparing an IL-6 receptor partial peptide using genetic engineering methods, a DNA sequence encoding the desired peptide is inserted into an expression vector, and then the peptide can be obtained by applying the aforementioned methods for expressing, producing, and purifying recombinant antibodies.

When producing an IL-6 receptor partial peptide by peptide synthesis methods, generally used peptide synthesis methods, for example, solid phase synthesis methods or liquid phase synthesis methods may be used.

Specifically, the peptides can be synthesized according to the method described in "Continuation of Development of Pharmaceuticals, Vol. 14, Peptide Synthesis (in Japanese) (ed. Haruaki Yajima, 1991, Hirokawa Shoten)". As a solid phase synthesis method, for example, the following method is used: the amino acid corresponding to the C terminus of the peptide to be synthesized is bound to a support that is insoluble in organic solvents, then the peptide strand is elongated by alternately repeating the reaction of condensing amino acids, whose α-amino groups and branch chain functional groups are protected with appropriate protecting groups, one at a time in a C- to N-terminal direction; and the reaction of removing the protecting groups from the α-amino groups of the resin-bound amino acids or peptides. Solid phase peptide synthesis is broadly classified into the Boc method and the Fmoc method, depending on the type of protecting groups used.

After synthesizing a protein of interest as mentioned above, deprotection reactions are carried out, then the peptide strand is cleaved from its support. For the cleavage reaction of the peptide strand, hydrogen fluoride or trifluoromethane sulfonic acid is generally used for the Boc method, and TFA is generally used for the Fmoc method. In the Boc method, for example, the above-mentioned protected peptide resin is treated with hydrogen fluoride in the presence of anisole. Then, the peptide is recovered by removing the protecting groups and cleaving the peptide from its support. By freeze-drying the recovered peptide, a crude peptide can be obtained. In the Fmoc method, on the other hand, the deprotection reaction and the reaction to cleave the peptide strand from the support can be performed in TFA using a method similar to those described above, for example.

Obtained crude peptides can be separated and/or purified by applying HPLC. Elution may be performed under optimum conditions using a water/acetonitrile solvent system, which is generally used for protein purification. The fractions corresponding to the peaks of the obtained chromatographic profile are collected and freeze-dried. Thus, purified peptide fractions are identified using molecular weight analysis by mass spectrum analysis, amino acid composition analysis, amino acid sequence analysis, or such.

Inhibitors of cancer metastasis as provided herein can be used for suppressing metastasis of cancer (for example, cancer cells) derived from a certain site, tissue, or organ to another site, tissue, or organ.

In the present invention, "cancer metastasis" refers to the phenomenon in which cancer derived from a certain site, tissue, or organ reaches another site, tissue, or organ, and proliferates to produce secondary tumors. In the present invention, "inhibition of cancer metastasis" means inhibition of cancer to metastasize to another site, tissue, or organ; decrease of the ratio of cancer metastasis to another site, tissue, or organ; prolongation of the time until cancer metastasizes to another site, tissue, or organ; and such.

Inhibitors of cancer metastasis may be used for suppressing metastasis of metastatic cancers such as colorectal cancer, breast cancer, lung cancer, prostate cancer, pancreatic cancer, and kidney cancer.

Preferably, suppression of cancer metastasis includes suppression of liver metastasis of cancer derived from a site, tissue, or organ other than the liver. In the present invention, cancer that metastasizes to the liver may be, without particular limitation, cancer derived from any site, tissue, or organ. Examples of primary foci include lung, breast, skin, colorectum, kidney, prostate, and pancreas. Metastasis inhibitors as provided herein can suppress, for example, metastasis of lung cancer, colon cancer, and such to the liver. Preferably, the suppression includes suppression of metastasis of lung cancer to the liver.

The effect of IL-6 inhibitors as metastasis inhibitors can be assessed, for example, using signal transduction-inhibiting activity as an indicator; however, the assessment is not limited thereto. The signal transduction-inhibiting activity of an IL-6 inhibitor can be evaluated by conventionally used methods. Specifically, the IL-6-dependent human myeloma lines S6B45 and KPMM2, human Lennert T lymphoma cell line KT3, or IL-6-dependent MH60.BSF2 cells are cultured, and IL-6 is added thereto. In the co-presence of an IL-6 inhibitor, ³H-thymidine uptake by the IL-6-dependent cells can be measured. Alternatively, IL-6 receptor-expressing U266 cells are cultured, and ¹²⁵I-labeled IL-6 is added thereto, and an IL-6 inhibitor is simultaneously added, then ¹²⁵I-labeled IL-6 bound to the IL-6 receptor-expressing cells is determined. In the above assay systems, the IL-6 inhibitory activity of an IL-6 inhibitor can be evaluated by including in addition to the IL-6 inhibitor-containing group, a negative control group that does not contain an IL-6 inhibitor, and comparing the results obtained from the two groups.

As shown in the Examples below, since an anti-IL-6 receptor antibody suppressed metastasis of cancer cells to the liver, IL-6 inhibitors such as anti-IL-6 receptor antibodies were shown to be useful as inhibitors of cancer metastasis to the liver.

Subjects to which a metastasis inhibitor provided herein is administered are mammals. The mammals are preferably humans.

The metastasis inhibitors provided herein can be administered as pharmaceuticals, and may be administered systemically or locally by oral or parenteral administration. For example, intravenous injections such as drip infusions, intramuscular injections, intraperitoneal injections, subcutaneous injections, suppositories, enemas, oral enteric tablets, or the like can be selected. Appropriate administration methods can be selected depending on the patient's age and symptoms. The effective dosage per administration is selected from the range of 0.01 to 100 mg/kg body weight. Alternatively, the dosage may be selected from the range of 1 to 1000 mg/patient, preferably from the range of 5 to 50 mg/patient. A preferred dosage and administration method are as follows. For example, when an anti-IL-6 receptor antibody is used, the effective dosage is an amount such that free antibody is present in the blood. Specifically, a dosage of 0.5 to 40 mg/kg body weight/month (four weeks), preferably 1 to 20 mg/kg body weight/month is administered by intravenous injection such as drip infusion, subcutaneous injection or such, once to several times a month, for example, twice a week, once a week, once every two weeks, or once every four weeks. The administration schedule may be adjusted by, for example, extending the administration interval of twice a week or once a week to once every two weeks, once every three weeks, or once every four weeks, while monitoring the condition of the patient and changes in the blood test values.

The inhibitors of liver cancer metastasis provided herein may contain pharmaceutically acceptable carriers such as preservatives and stabilizers. "Pharmaceutically acceptable carrier" refers to a material that can be administered in combination with the above agents. Such pharmaceutically acceptable materials include, for example, sterile water, physiological saline, stabilizers, excipients, buffers, preservatives, detergents, chelating agents (EDTA and such), and binders.

Herein, detergents include non-ionic detergents, and typical examples include sorbitan fatty acid esters such as sorbitan monocaprylate, sorbitan monolaurate, and sorbitan monopalmitate; glycerin fatty acid esters such as glycerin monocaprylate, glycerin monomyristate and glycerin monostearate; polyglycerin fatty acid esters such as decaglyceryl monostearate, decaglyceryl distearate, and decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, and polyoxyethylene sorbitan tristearate; polyoxyethylene sorbit fatty acid esters such as polyoxyethylene sorbit tetrastearate and polyoxyethylene sorbit tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol, polyoxyethylene polyoxypropylene propyl ether, and polyoxyethylene polyoxypropylene cetyl ether; polyoxyethylene alkyl phenyl ethers such as polyoxyethylene nonylphenyl ether; polyoxyethylene hardened castor oils such as polyoxyethylene castor oil and polyoxyethylene hardened castor oil (polyoxyethylene hydrogenated castor oil); polyoxyethylene beeswax derivatives such as polyoxyethylene sorbit beeswax; polyoxyethylene lanolin derivatives such as polyoxyethylene lanolin; and polyoxyethylene fatty acid amides and such with an HLB of six to 18, such as polyoxyethylene stearic acid amide.

Detergents also include anionic detergents, and typical examples include, for example, alkylsulfates having an alkyl group with ten to 18 carbon atoms, such as sodium cetylsulfate, sodium laurylsulfate, and sodium oleylsulfate; polyoxyethylene alkyl ether sulfates in which the alkyl group has ten to 18 carbon atoms and the average molar number of added ethylene oxide is 2 to 4, such as sodium polyoxyethylene lauryl sulfate; alkyl sulfosuccinate ester salts having an alkyl group with eight to 18 carbon atoms, such as sodium lauryl sulfosuccinate ester; natural detergents, for example, lecithin; glycerophospholipids; sphingo-phospholipids such as sphingomyelin; and sucrose fatty acid esters in which the fatty acids have 12 to 18 carbon atoms.

One, two or more of the detergents described above can be combined and added to the agents provided herein. Detergents that are preferably used in the preparations of the present disclosure include polyoxyethylene sorbitan fatty acid esters, such as polysorbates 20, 40, 60, and 80. Polysorbates 20 and 80 are particularly preferred. Polyoxyethylene polyoxypropylene glycols, such as poloxamer (Pluronic F-68^{®} and such), are also preferred.

The amount of detergent added varies depending on the type of detergent used. When polysorbate 20 or 80 is used, the amount is generally in the range of 0.001 to 100 mg/ml, preferably in the range of 0.003 to 50 mg/ml, more preferably in the range of 0.005 to 2 mg/ml.

Herein, buffers include phosphate, citrate buffer, acetic acid, malic acid, tartaric acid, succinic acid, lactic acid, potassium phosphate, gluconic acid, capric acid, deoxycholic acid, salicylic acid, triethanolamine, fumaric acid, and other organic acids; and carbonic acid buffer, Tris buffer, histidine buffer, and imidazole buffer.

Liquid preparations may be formulated by dissolving the agents in aqueous buffers known in the field of liquid preparations. The buffer concentration is generally in the range of 1 to 500 mM, preferably in the range of 5 to 100 mM, more preferably in the range of 10 to 20 mM.

The agents provided herein may also comprise other low-molecular-weight polypeptides; proteins such as serum albumin, gelatin, and immunoglobulin; amino acids; sugars and carbohydrates such as polysaccharides and monosaccharides, sugar alcohols, and such.

Herein, amino acids include basic amino acids, for example, arginine, lysine, histidine, and ornithine, and inorganic salts of these amino acids (preferably hydrochloride salts, and phosphate salts, namely phosphate amino acids). When free amino acids are used, pH is adjusted to a preferred value by adding appropriate physiologically acceptable buffering substances, for example, inorganic acids, and in particular hydrochloric acid, phosphoric acid, sulfuric acid, acetic acid, and formic acid, and salts thereof. In this case, the use of phosphate is particularly beneficial because it gives quite stable freeze-dried products. Phosphate is particularly advantageous when preparations do not substantially contain organic acids, such as malic acid, tartaric acid, citric acid, succinic acid, and fumaric acid, or do not contain corresponding anions (malate ion, tartrate ion, citrate ion, succinate ion, fumarate ion, and such). Preferred amino acids are arginine, lysine, histidine, and ornithine. Acidic amino acids can also be used, for example, glutamic acid and aspartic acid, and salts thereof (preferably sodium salts); neutral amino acids, for example, isoleucine, leucine, glycine, serine, threonine, valine, methionine, cysteine, and alanine; and aromatic amino acids, for example, phenylalanine, tyrosine, tryptophan, and its derivative, N-acetyl tryptophan.

Herein, sugars and carbohydrates such as polysaccharides and monosaccharides include, for example, dextran, glucose, fructose, lactose, xylose, mannose, maltose, sucrose, trehalose, and raffinose.

Herein, sugar alcohols include, for example, mannitol, sorbitol, and inositol.

When the agents provided herein are prepared as aqueous solutions for injection, the agents may be mixed with, for example, physiological saline, and/or isotonic solution containing glucose or other auxiliary agents (such as D-sorbitol, D-mannose, D-mannitol, and sodium chloride). The aqueous solutions may be used in combination with appropriate solubilizing agents such as alcohols (ethanol and such), polyalcohols (propylene glycol, PEG, and such), or non-ionic detergents (Polysorbate 80 and HCO-50).

If desired, the agents may further comprise diluents, solubilizers, pH adjusters, soothing agents, sulfur-containing reducing agents, antioxidants, and such.

Herein, the sulfur-containing reducing agents include, for example, compounds comprising sulfhydryl groups such as N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and thioalkanoic acids having one to seven carbon atoms.

Moreover, herein the antioxidants include, for example, erythorbic acid, dibutylhydroxy toluene, butylhydroxy anisole, α-tocopherol, tocopherol acetate, L-ascorbic acid and salts thereof, L-ascorbic acid palmitate, L-ascorbic acid stearate, sodium hydrogen sulfite, sodium sulfite, triamyl gallate, propyl gallate, and chelating agents such as disodium ethylenediamine tetraacetate (EDTA), sodium pyrophosphate, and sodium metaphosphate.

If required, the agents may be encapsulated in microcapsules (microcapsules of hydroxymethylcellulose, gelatin, poly[methylmethacrylic acid] or such) or prepared as colloidal drug delivery systems (liposome, albumin microspheres, microemulsion, nano-particles, nano-capsules, and such) (see "Remington's Pharmaceutical Science 16th edition", Oslo Ed., 1980, and the like). Furthermore, methods for preparing agents as sustained-release agents are also known, and are applicable to the present disclosure (Langer et al., J. Biomed. Mater. Res. 1981, 15: 167-277; Langer, Chem. Tech. 1982, 12: 98-105; U.S. Patent No. 3,773,919; European Patent Application No. (EP) 58,481; Sidman et al., Biopolymers 1983, 22: 547-556; and EP 133,988).

Pharmaceutically acceptable carriers used are appropriately selected from those described above or combined depending on the type of dosage form, but are not limited thereto.

The present disclosure relates to methods for suppressing metastasis of cancer to another site, tissue, or organ in a subject, which comprise the step of administering an IL-6 inhibitor to a subject who has developed cancer.

In the present disclosure, "subject" refers to an organism to which a metastasis inhibitor of the present disclosure is administered, or a portion of the body of the organism. The organisms include animals (for example, humans, domestic animal species, and wild animals), but are not particularly limited thereto.

Furthermore, there is no particular limitation on the sites, tissues, or organs in which metastasis occurs; however, a preferred example is metastasis to the liver. A particularly preferred example is metastasis of lung cancer to the liver.

In the present disclosure, "administration" includes oral and parenteral administration. Oral administration includes administration in the form of oral agents. Dosage forms such as granules, powders, tablets, capsules, solvents, emulsions, and suspensions can be selected for oral agents.

Parenteral administration includes, for example, administration in the form of injections. Examples of such injections include subcutaneous injections, intramuscular injections, and intraperitoneal injections. Meanwhile, the effects of the methods provided herein can be achieved by introducing a gene comprising an oligonucleotide to be administered into a living body using a gene therapy technique. Alternatively, a pharmaceutical agent as provided herein may be administered locally to the site to be treated. For example, the agent can be administered by local injection during surgery, use of a catheter, or targeted gene delivery of a DNA encoding a peptide disclosed herein.

When carrying out the methods of the present disclosure, a pharmaceutical agent provided herein may be administered as part of a pharmaceutical composition together with at least one known pharmaceutical agent. Alternatively, a pharmaceutical agent provided herein may be administered simultaneously with at least one known anticancer agent (for example, inhibitor of cancer metastasis). In an embodiment, a pharmaceutical agent provided herein and a known anticancer agent may be administered substantially simultaneously.

Furthermore, the present disclosure relates to methods of screening for substances that suppress cancer metastasis, which comprise the steps of:
(a) determining the IL-6-inhibiting activity of a test substance; and
(b) selecting a substance that has IL-6-inhibiting activity.

The IL-6-inhibiting activity can be determined by methods known to those skilled in the art. For example, the IL-6-inhibiting activity of a test substance can be measured by the above-mentioned methods.

Substances obtained by the screening of the present disclosure may be used to suppress cancer metastasis, in particular, metastasis to the liver.

### Examples

Hereinbelow, the present disclosure will be specifically described with reference to the Examples, but it is not to be construed as being limited thereto.

### [Materials and methods]

### <Animals>

Ikkβ^{F/F} mice, Ikkβ^{F/F}:Alb-Cre mice (referred to as Ikkβ^{Δhep}), and Ikkβ^{F/F}:Mx1-Cre mice (referred to as "Ikkβ^{L+H}" after poly (IC) injection) are as described in the literature (Maeda, S., et al. Immunity 19, 725-737 (2003); and Hsu, L.C., et al. Nature 428, 341-345 (2004)). All mice were backcrossed to C57BL/6 at least ten times. Ikkβ^{+/+}:Alb-Cre mice, Ikkβ^{+/+}:Mx1-Cre mice, IL-6 knockout (IL-6 KO) mice, IL-1 receptor knockout (IL-1 RKO) mice, and C57BL/6 wild-type (WT) mice were purchased from the Jackson Laboratory. All mice were bred in a cage equipped with a filter on top, and given autoclave-sterilized feed and water according to the NIH guidelines at University of California San Diego (UCSD) and Institute for Adult Diseases, Asahi Life Foundation.

### <Induction and analysis of metastatic tumors>

LLC and B16F10 cells (500,000 cells/animal) were suspended in 100 µL of phosphate-buffered saline solution (PBS) and injected into the spleen of 6 to 8 week-old anesthetized mice. The cells were allowed to move into the liver for a few minutes, and then the spleen was removed. All mice recovered satisfactorily after the operation, and their physical conditions were monitored daily. After eleven days, most of the animals showed discomfort, and they were immediately sacrificed by CO₂ asphyxia. Tumors observed on the external surface of the liver (≥ 0.5 mm) were counted and measured using a stereomicroscope.

### <Antibodies and chemical substances>

The following antibodies were used: anti-IκBα antibody, anti-phosphorylated IκBα antibody, anti-STAT3 antibody, and anti-phosphorylated STAT3 antibody (Cell Signaling Biotechnology); anti-TF2D antibody and anti-PCNA antibody (Santa Cruz Biotechnology); anti-F4/80 antibody (Caltag); von Willebrand factor (vWF; Wako); and anti-IL-6 antibody (R&D Systems). A neutralizing anti-IL-6 receptor antibody was provided by Chugai Pharmaceutical Co. Ltd. (Becker, C., et al. Immunity 21, 491-501 (2004)). The NEMO-binding domain peptide is described in the literature (Shibata, W., et al. J. Immunol. 179, 2681-2685 (2007)). The mouse group was treated with NBD or a mutated (mut) NBD peptide at a dose of 4 mg/kg by intraperitoneal injection.

### <Cells>

Mouse Lewis lung cancer (LLC) cells were maintained in Dulbecco's modified Eagle medium (DMEM) containing 10% fetal bovine serum (FBS). The mouse macrophage cell line J774A.1 was maintained in an RPMI medium containing 10% FBS.

### <Isolation of cultured primary macrophages and nonparenchymal cells>

Bone marrow-derived macrophages (BMDM) were cultured according to literature (Hsu, L.C., et al. Nature 428, 341-345 (2004)). Nonparenchymal (NP) cells of the liver were isolated by collagenase digestion and differential centrifugation. The liver was perfused *in situ* as described in literature (Maeda, S., et al. Immunity 19, 725-737 (2003)). The cell suspension was filtered through nylon gauze, and liver cells were removed by centrifuging the filtrate twice at 50 x g for one minute. The NP fraction was washed with buffer, and then the cells were seeded onto a plastic culture plate and cultured for one hour.

### <Biochemical and Immunohistochemical analyses>

Protein lysates were prepared from tissues and cultured macrophages, and separated by SDS-polyacrylamide gel electrophoresis (PAGE). Then, the proteins were transferred onto an Immobilon membrane (Millipore), and analyzed by immunoblotting. Total cellular RNA was extracted using the TRIZOL reagent (Invitrogen), and cDNA was synthesized using Superscript II (Invitrogen). Specific mRNA expression was quantified using real-time polymerase chain reaction (PCR), and this was normalized against GAPDH mRNA expression. Suitable primer sequences can be used. For array analysis, the mouse NF-κB Signaling Pathway PCR Array (SABiosciences) was used according to the manufacturer's instructions.

Electrophoretic mobility shift assay (EMSA) was performed as described in literature (Maeda, S., et al. Immunity 19, 725-737 (2003)). The levels of cytokine and soluble IL-6 receptor (IL-6sR) were determined using enzyme-linked immunosorbent assay (ELISA) (R&D System).

Liver tissues were fixed in 10% formaldehyde, and then dehydrated and embedded in paraffin to produce sections (5 µm-thick). The paraffin in the sections was removed, and rehydration was performed. Then, the sections were treated with a PBS solution containing 3% H₂O₂, and then incubated with a suitable antibody. The binding of the primary antibody was detected using a biotinylated secondary antibody (1:500 dilution; Vector Laboratories), and streptavidin-horseradish peroxidase reaction was performed. Visualization was carried out with 3,3'-diaminobenzidine (DAB; Sigma), and counterstaining was carried out using hematoxylin.

For immunofluorescent staining, frozen sections were incubated with an appropriate antibody at 4°C overnight, and then visualized using secondary antibodies labeled with Alexa Fluor 488 and 555 (Molecular Probes).

### <Analysis of cell viability and cell cycle by flow cytometry>

Viable cells were measured at 450 nm utilizing Cell Counting Kit-8 (Dojindo Molecular Tech) which uses [2-(2-methoxy-4-nitrophenyl)-3-(4-nitrophenyl)-5-(2,4-disulfophenyl)-2H-tetrazolium, moriosodium salt].

Cell populations at G0/G1, S, and G2-M were determined by flow cytometry analysis of DNA contents. The values for the cell cycle were each represented by the average of three measured values, and the sub-G1 stage value represents the percentage of cells at sub-G1 to the total number of cells in the cell cycle and was shown as mean ± SE.

### <Statistical analysis>

Data are represented as the mean ± standard error of the mean (SEM). The significant difference was detected using Student's t-test. P values of ≤ 0.05 were considered significant. In all cases, the size of the group was selected to produce statistically clear results.

### [Example 1] NF-κB activation in tumor cells does not affect tumor metastasis

To investigate the action of NF-κB activation on tumor metastasis, the present inventors injected Lewis lung cancer (LLC) cells, which have high metastatic potential, into the mouse liver through the spleen. Electrophoretic mobility shift assay (EMSA) determined that LLC inoculation activates NF-κB in the liver (Fig. 1A). Immunostaining of phospho-IκBα, which is a marker of NF-κB activation, was also observed in the liver four hours after LLC inoculation (Fig. 1B). Staining with F4/80, which is a marker of Kupffer cells or macrophages, showed that anti-phospho-IκBα staining (i.e., NF-κB activation) occurs mainly in Kupffer cells (Fig. 1B). NF-κB activation was not observed in PBS-injected (sham-operated) mice (Fig. 1C).

To investigate the role of NF-κB activation in liver metastasis, the present inventors stably expressed in LLC cells an undegradable IκBα protein, which is a mutant with substitutions of Ser³² and Ser³⁶ with alanine. This protein is known as the IκB super repressor (s-rIκB), and blocks the classical NF-κB pathway. The NF-κB activity induced by TNFα was more strongly inhibited in s-rIκB-transfected cells (LLC/SR) than in cells transfected with an empty vector (LLC/M) (Fig. 2A). However, there was no difference in tumor growth in vitro between LLC/M and LLC/SR cells (Fig. 2B).

Eleven days after administration of LLC cells into the spleen, liver metastasis was induced. At this time, it was possible to accurately measure the tumor number and the tumor-occupied area (Fig. 3B). Male wild-type mice (WT) were inoculated with LLC/M or LLC/SR. After eleven days, there was no significant difference in the tumor number in the liver between the LLC/M-inoculated mice and LLC/SR-inoculated mice (Fig. 3A). These results indicate that NF-κB activation in LLC cells did not affect liver metastasis in this model.

### [Example 2] NF-κB activation in nonparenchymal cells is essential for tumor metastasis

To investigate the role of NF-κB activation in mouse liver metastasis, LLC cells were injected into the spleen of male mice which are homozygous for either a liver cell-specific IKKβ deletion (Ikkβ^{Δhep}) or floxed Ikkβ allele (Ikkβ^{F/F}) in which a portion of the target gene has been deleted (Maeda, S., et al. Immunity 19, 725-737 (2003)). In Ikkβ^{Δhep} mice, IKKβ which is essential for NF-κB activation was absent from liver cells, but present in nonparenchymal cells (NPs) (Maeda S., et al. Cell 2005;121:977-990). The tumor number and the tumor-occupied area were not significantly different among Ikkβ^{F/F} mice, Ikkβ^{+/+}:Alb-cre mice, and Ikkβ^{Δhep} mice (Figs. 4A and 4B). This suggests that the presence of IKKβ in liver cells did not affect metastasis of tumors induced by LLC cells. To examine the role of NP in metastasis, the present inventors crossed Ikkβ^{F/F} mice with Mx-1-Cre transgenic mice which express Cre recombinase under the interferon-inducible Mx1 promoter. When poly (IC) which induces interferon production is injected into Ikkβ^{F/F}:Mx-1-Cre mice, IKKβ is efficiently deleted from the liver and spleen; however, IKKβ is not deleted from most of the other tissues. Deletion by Mx-1-Cre is very effective in lymphocytes, Kupffer cells, and liver cells in addition to macrophages (Hsu LC., et al. Nature 2004;428:341-345). Whole liver IKKβ knockout mice (Ikkβ^{ΔL+H}) showed liver metastasis at a significantly lower liver weight, fewer metastatic foci, and smaller tumor-occupied area than poly (IC)-injected Ikkβ^{F/F} mice and Ikkβ^{+/+}:Mx-1-Cre mice (Figs 4C and 4D, and data not shown). The liver of Ikkβ^{F/F} mice was markedly swollen compared to the liver of Ikkβ^{ΔL+H} mice (Fig. 4E). Histopathological analysis of liver tissues collected from Ikkβ^{F/F} mice (Fig. 4E) showed significant tumor growth with an extensive adhesive region of tumor cells in which atypical nuclei, large number of dividing cells, and central region of necrosis and bleeding are present. In contrast, the liver derived from Ikkβ^{ΔL+H} mice showed tumor growth with low aggressiveness in which multiple focal regions with low tumor invasion are dispersed randomly throughout the entire hepatic parenchyma (Fig. 4E). To determine whether or not this decrease of tumor metastasis in Ikkβ^{ΔL+H} mice is tumor cell type-specific, the present inventors used the melanoma cell line B16F10. After injection of B16F10, tumor metastasis was inhibited in Ikkβ^{ΔL+K} mice (Fig. 4F). This suggests that liver NP, rather than liver cells, is the cell type essential for liver metastasis.

### [Example 3] Ikkβ^{ΔL+H}. mice express a relatively low level of IL-6, and IL-6 deletion decreases metastatic tumor

Next, the present inventors investigated gene expression regulated by NF-κB in the LLC-injected liver and sham-operated liver using a real-time PCR array. The present inventors discovered that the mRNA expression of several genes was up-regulated by LLC injection into WT mice (Tables 1 and 2). The present inventors compared the expression levels of IL-1β, IL-6, and TNFα which were up-regulated in the array analysis of Ikkβ^{F/F}, Ikkβ^{Δhep}, and Ikkβ^{ΔL+H} mice. It was found that when tumors were injected into the spleen, the mRNA expression of IL-1β and IL-6 was induced in Ikkβ^{F/F} and Ikkβ^{Δhep} mice, but their expression was relatively low in Ikkβ^{ΔL+H} mice (Fig. 5A and data not shown). No difference was observed in the expression of TNFα mRNA in the liver before and after LLC injection in all of the strains (Fig. 5A and data not shown). Furthermore, the present inventors analyzed the mRNA expression of COX-2 and MMP-9 which are regulated by NF-κB and associated with metastasis, and discovered that the expression of these genes is also relatively low in Ikkβ^{ΔL+H} mice (Fig. 5A). IL-1β and IL-6 are major factors for inflammatory response, and are considered to function as tumor promoting factors (Vidal-Vanaclocha, F., et al., J. Natl. Cancer Inst. 88, 198-205 (1996); and Aggarwal, B.B., et al., Biochem. Pharmacol. 72, 1605-1621 (2006)). To determine whether or not IL-1β or IL-6 is related to tumor metastasis, the present inventors used IL-1 receptor knockout (IL-1 RKO) mice or IL-6 knockout (IL-6 KO) mice. The number of metastatic tumors was slightly decreased in IL-1 RKO mice compared to the WT control, but the difference was not significant. On the other hand, IL-6 KO mice showed a significant decrease in the tumor number (Fig. 5B). IL-6 was expressed 12 hours after LLC injection, and seemed to be localized mainly in anti-F4/80-positive Kupffer cells (Fig. 5C). IL-6 induces STAT3 phosphorylation, and regulates the expression of STAT dependent genes (Zhong, Z., et al., Science 264, 95-98 (1994)). In this Example, LLC injection caused STAT3 phosphorylation in the liver 8 to 12 hours after injection, and thereafter, the phosphorylation was decreased 24 hours after injection (Fig. 5D). STAT3 phosphorylation was markedly decreased in IL-6 KO mice as expected (Fig. 5D).

**[Table 1]**

| Genes up-regulated by LLC injection | | |
|---|---|---|
| Gene symbol | Gene name | Ratio (LLC/sham) |
| N1rp12 | NLR family, pyrin domain containing 12 | 18.2 |
| Egrl | Early growth response 1 | 13.2 |
| I16 | Interleukin 6 | 7.5 |
| Tnf | Tumor necrosis factor | 6.5 |
| Ccl2 | Chemokine (C-C motif) ligand 2 | 6.3 |
| Tnfsf14 | Tumor necrosis factor (ligand) superfamily, member 14 | 6.1 |
| Csf2 | Colony stimulating factor 2 (granulocyte-macrophage) | 3.5 |
| I11b | Interleukin 1 beta | 3.1 |
| Tnfaip3 | Tumor necrosis factor, alpha-induced protein 3 (A20) | 2.8 |
| Ifng | Interferon gamma | 2.6 |

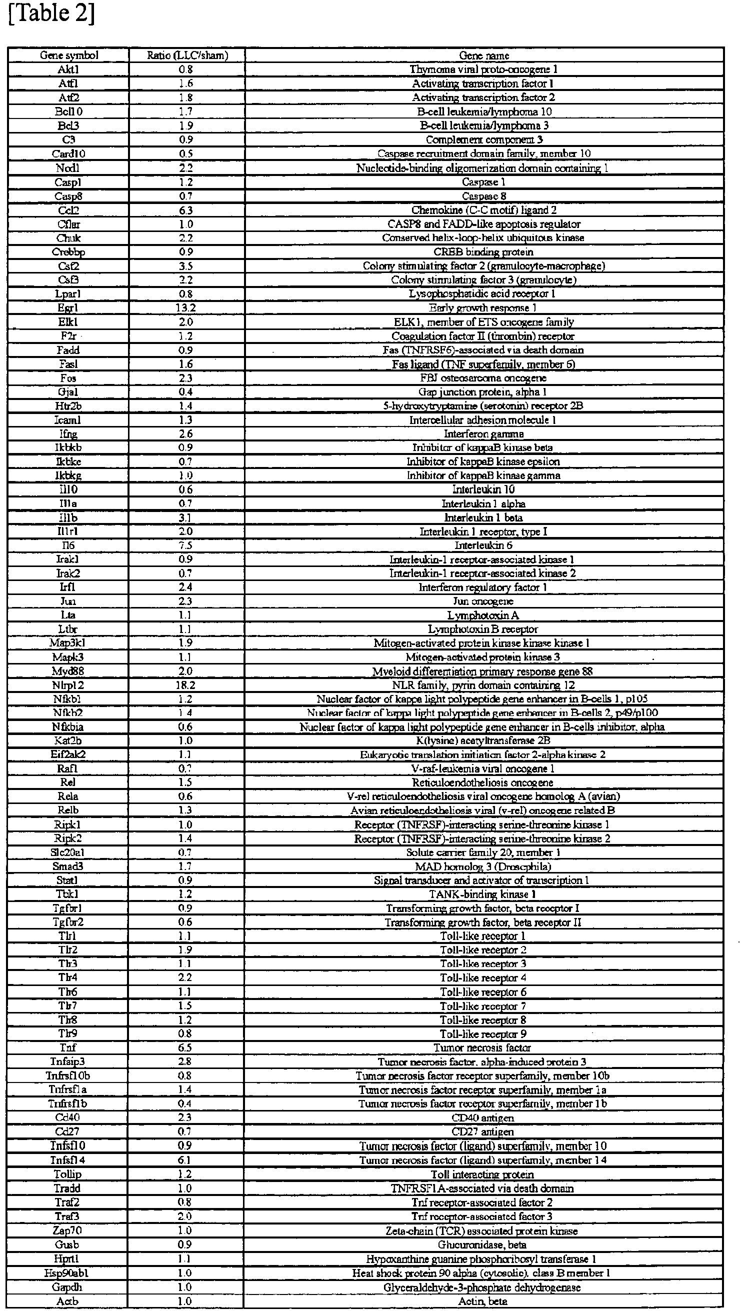

### [Example 4] IL-6 enhances tumor angiogenesis by VEGF expression

The present inventors predicted that IL-6 production occurs after LLC injection in Kupffer cells which are indigenous hepatic macrophages. To prove the involvement of Kupffer cells, GdCl₃ was injected into WT mice to deplete Kupffer cells as described in the literature (Maeda S., et al. Cell 2005;121:977-990). After 48 hours, LLC cells were injected into the spleen, and 11 days later, the mice were tested for tumor load. GdCl₃-injected mice produced liver metastasis with significantly smaller metastatic foci (25.1 ±4.8 versus 11.8 ± 2.5, P < 0.05) compared to solvent-treated mice. These results suggest that Kupffer cells are critically important for tumor metastasis.

Next, the present inventors used primary cultured macrophages derived from Ikkβ^{F/F} and Ikkβ^{ΔL+H} mice to analyze the direct action of LLC cells. When determined by IκBα degradation, an LLC culture supernatant induced NF-κB activation in Ikkβ^{F/F} macrophages. However, NF-κB activation was not observed in Ikkβ^{ΔL+H} macrophages (Fig. 6A). Both LLC culture supernatant and LPS treatments induced IKKβ-dependent IL-6 production in primary culture macrophages (Fig. 6B). These results suggest that LLC cells secrete a factor that activates IKK/NF-κB and induces IL-6 production.

After appearance of metastatic tumors on day 11, the serum IL-6 concentration was increased (Fig. 6C). IL-6 has been reported to be involved in cell growth and anti-apoptosis signal transduction in tumorigenesis (Wehbe, H., et al., Cancer Res. 66, 10517-10524 (2006)). To test this possibility, LLC cells were stimulated with IL-6 and subjected to cell cycle analysis. It was found that the rate of transition from the G2 to M phase was increased from 23.4 ± 2.5% to 29.4 ± 2.3% (p < 0.05), and the percentage of the sub-G1 population (i.e., apoptotic cells) was decreased from 9.9 ± 1.5% to 1.7 ± 1.2% (p < 0.05). To evaluate the effect of IL-6 on cell growth, the present inventors determined the cell count at several time points, and showed that LLC cell growth was significantly increased by IL-6 treatment (Fig. 6D). These results suggest that IL-6 promotes the establishment of metastatic tumors by promoting cell growth and inhibiting apoptosis.

The present inventors analyzed tumor cell growth by PCNA staining of metastatic tumors, and discovered that Ikkβ^{ΔL+H} and IL-6 KO mice show fewer PCNA-positive cells compared to the control (Figs. 6E and 6F).

Furthermore, the present inventors analyzed the effect of IL-6 in B16F10 cells. Injection of B16F10 cells induced IL-6 production (as well as IL-1β production) in WT mice; on the other hand, the induction was less stimulated in Ikkβ^{ΔL+H} mice in a manner similar to the action of LLC cells (Fig. 7A). In addition, injection of B16F10 cells activated STAT3 in the liver of WT mice (Fig. 7B). STAT3 was activated by IL-6 treatment in vitro, and cell growth was significantly increased (Figs. 7C and 7D).

### [Example 5] IL-6 promotes tumor vasculogenesis by VEGF expression

The use of angiogenesis inhibitors for treatment of metastasis is clearly very effective for particular cancers. Metastatic tumors derived from injected LLC also depend on neovascularization (Lee HJ, et al., Carcinogenesis 2006;27:2455-2463), and IL-6 is involved in neovascularization through VEGF expression (Loeffler S, et al., Int J Cancer 2005;115:202-213). In this Example, immunostaining for the von Willebrand factor (vWF) which is a blood glycoprotein showed that tumor angiogenesis was increased in metastatic liver tumors (Fig. 8A). Compared to the control, angiogenesis was decreased in metastatic tumors of Ikkβ^{ΔL+H} and IL-6 KO mice (Fig. 8B).

To determine whether or not IL-6 induces VEGF production, liver NP and mouse embryonic fibroblasts (MEF) were treated with IL-6 in the presence or absence of a soluble IL-6 receptor (IL-6sR). ELISA test results showed that IL-6, when used in combination with IL-6sR, induced VEGF production in NP and MEF (Fig. 8C). After appearance of metastatic tumors on day 11, the serum VEGF concentration was increased in WT mice, whereas VEGF was not sufficiently expressed in IL-6KO mice (Fig. 8D). The present inventors determined the IL-6sR concentration in the sera of LLC-injected and non-LLC-injected mice, and discovered that IL-6R was abundantly expressed in all animals regardless of the LLC injection (Fig. 8E).

To investigate whether or not this finding is clinically applicable, the present inventors treated LLC cells with IL-6 in the presence or absence of a neutralizing anti-IL-6 receptor antibody (Hsu LC, et al., Nature 2004;428:341-345). Simultaneous treatment with IL-6 and the antibody inhibited STAT3 phosphorylation in LLC cells (Fig. 8G). Next, the present inventors inoculated wild-type mice with LLC cells, and anti-IL-6 was administered on days 0, 3, 6, and 9 after LLC injection. On day 11 post-LLC injection, the number of metastatic tumors was decrease by 50% at maximum in mice treated with anti-IL-6 (Fig. 8F).

Furthermore, the present inventors performed a supplementary experiment by administering IL-6 (1 mg/mouse) and IL6R (1 mg/mouse) to Ikkβ^{ΔL+H}. The tumor number was increased slightly [Ikkβ^{ΔL+H}: 4.1 ± 1.1 (n = 9), Ikkβ^{ΔL+H} + IL-6/IL-6R: 8.4 ± 1.9 (n = 5) (P = 0.045)]. These results suggest that IL-6 is important for development of liver metastasis, but other factors such as TNFα, IL-1, and MMP may induce metastasis in cooperation with IL-6. In other words, the presence of IL-6 alone is not a sufficient condition, but is a necessary condition for cancer metastasis.

### [Example 6] The NEMO-binding domain peptide inhibits tumor metastasis

The present inventors investigated whether the NBD peptide, which inhibits NF-κB activity by blocking association between NEMO and IKKβ (May, M.J., et al. Science 289, 1550-1554 (2000)), decreases tumor metastasis. NBD treatment inhibited NF-κB activation (Fig. 9A) and IL-6 induction (data not shown) which are induced by an LLC supernatant or LPS in J774.1 mouse macrophage cells. The present inventors discovered that when NBD peptide treatment is performed 0, 3, 6, and 9 days after LLC injection, NBD-treated mice with liver metastasis showed a significantly lower liver weight and fewer metastatic foci compared to the control mice (Figs. 9B and 9C), but a mutant NBD peptide did not show any effect (Figs. 9B and 9C).

### [Discussion]

The present disclosure proves that IL-6 is an essential regulatory factor for liver metastasis. Furthermore, the present disclosure indicates that IL-6 which is strongly related to NF-κB activation is also involved in tumor metastasis, and may become an important therapeutic target for treating liver metastasis.

Recently, it was reported that TLR2-dependent TNFα expression is necessary for lung metastasis, but IL-6 which is induced in a manner similar to TNFα is not involved (Kim, S., et al. Nature in press.). The reason why TNFα is important for lung metastasis and IL-6 is important for liver metastasis is unclear. The mechanisms for producing an inflammatory microenvironment are speculated to be different between lung and liver. In fact, IL-6 is particularly important for liver regeneration, and it is also essential for the development of liver cancer (Naugler, W.E., et al. Science 317, 121-124 (2007); and Cressman, D.E., et al. Science 274, 1379-1383 (1996)). In contrast, the involvement of TNFα in liver regeneration has been open to dispute (Hayashi, H., et al. Liver Int 25, 162-170 (2005); and Yamada, Y, et al. Proc Natl Acad Sci U S A 94, 1441-1446 (1997)), and TNFα is not essential for the onset of cancer (Naugler, W.E., et al. Science 317, 121-124 (2007)). Since LLC administration did not cause differences in the liver TNFα expression between Ikkβ^{F/F} mice and Ikkβ^{ΔL+H} mice, the present inventors did not analyze the involvement of TNFα expression.

The present inventors' results suggest that metastasis induced by NF-κB activation is related to IL-6-mediated angiogenesis and cell proliferation. Factors other than IL-6 are also thought to be related to NF-κB-activated tumor metastasis. For example, inhibition of NF-κB activation reduces MMP9 expression which is strongly related to tumor metastasis (Coussens, L.M. & Werb, Z. Nature 420, 860-867 (2002)). The present inventors discovered that MMP9 is activated in an IKKβ-dependent manner in the liver of LLC-injected mice (Fig. 10). Furthermore, COX-2 expression is also IKKβ-dependent, and this is consistent with a previous report (Chen, C.C., et al. Mol. Pharmacol. 59, 493-500 (2001)). COX-2-related angiogenesis and cell proliferation are thought to be involved in metastasis.

In contrast, the induction of many genes which are up-regulated by tumor cell injection in the control mice was relatively low in Ikkβ^{ΔL+H} mice, but it was not completely inhibited. This suggests that other factors, in addition to NF-κB, may be involved in the transcriptional up-regulation. For example, IL-6 expression is regulated by not only NF-κB but also AP-1 and C/EBP (Pritts T., et al. Am J Surg 2002;183:372-83). It is predicted that these markers may become other therapeutic targets for tumor metastasis.

There is still debate over whether inflammation promotes or inhibits metastasis. However, previous studies on tumor-associated macrophages (TAMs) and inflammation-associated carcinogenesis models (Luo, Y, et al. J. Clin. Invest. 116, 2132-2141 (2006); and Lewis, C.E. & Pollard, Cancer Res. 66, 605-612 (2006)) suggest that inflammation promotes the onset of cancer. The present inventors did not predict that the onset of metastatic liver tumor depends on inflammation. However, the present inventors' results indicate that IKKβ which is a major active substance for inflammatory response plays an important role in metastasis. On the other hand, anti-carcinogenic effects were obtained only when IKKβ was eliminated from Kupffer cells, other types of bone marrow cells, and liver cells. These results indicate that Kupffer cells are essential for mitogen production. In the models described herein, accumulation of macrophages was observed around and inside metastatic tumors. Since deletion of IKKβ decreased macrophage accumulation, the results by the present inventors suggest that tumor metastasis is associated with inflammatory response.

In Ikkβ^{ΔL+H} mice, IKKβ has been removed from not only liver cells and Kupffer cells but also other cells such as endothelial cells (Lee PY, et al. Arthritis Rheum. 2007;56:3759-69). In fact, NF-κB activation and IL-6 expression were mainly observed in Kupffer cells, and the present inventors found that relatively low liver metastasis was shown in mice depleted of Kupffer cells by injection of GdCl₃. This suggests that Kupffer cells are essential for the onset of metastasis.

The present inventors showed that in their model the NF-κB activity in LLC cells does not contribute to metastasis. Since the test used the expression of IκBαSR which inhibits the function of IκBα, the present inventors could not exclude the possibility that NF-κB activation in cancer cells might be related to metastasis. Cell proliferation decreases or increases depending on NF-κB (Chen, F., et al. J. Biol. Chem. 281, 37142-37149 (2006)). This suggests that the role of NF-κB activation in cell growth is cell type-specific. Furthermore, constitutive activation of NF-κB is sometimes observed in cancer cells, and such NF-κB-activated cells show strong metastatic activity (Fujioka, S., et al. Oncogene 22, 1365-1370 (2003); and Nakshatri, H., et al. Mol. Cell. Biol. 17, 3629-3639 (1997)). Thus, inhibition of the NF-κB pathway may be effective for delaying metastasis in specific cell types.

However, NF-κB inhibitors have problems in clinical practice such as side effects caused by modulation of the immune system (Karin, M., et. al., Nat Rev Drug Discov 3, 17-26 (2004)). The use of NF-κB inhibitors may have a particularly high risk for patients suffering from immunodeficiency. On the other hand, IL-6 inhibition is thought to be a promising approach for anticancer therapy. To date, IL-6 inhibition has been used clinically for several inflammatory diseases including rheumatoid arthritis and Castleman's disease (Sebba, A. Am. J. Health Syst. Pharm. 65, 1413-1418 (2008)). Based on the previous research results and the present inventors' results, it is thought that IL-6 inhibition can be clinically applicable to metastasis-associated cancers such as liver cancer, colorectal cancer, and breast cancer in the future.

In summary, the present inventors' results indicate that liver tumor metastasis depends on inflammation, and raise the hope for anti-inflammatory intervention targeting Kupffer cells in the chemical prevention of metastatic tumors. The present inventors' results prove that the IKKβ/NF-κB signal transduction pathway is an attractive target for the development of anti-metastatic agents.

### Industrial Applicability

The present disclosure demonstrates that cancer metastasis to the liver can be suppressed by administration of an anti-IL-6 receptor antibody. Therefore, IL-6 inhibitors of the present disclosure are useful as inhibitors of metastatic cancer.

## Claims

1. An agent for use in a method of inhibiting cancer metastasis comprising an interleukin-6 (IL-6) inhibitor as an active ingredient, wherein the IL-6 inhibitor is an anti-IL-6 receptor antibody.

2. Use of an IL-6 inhibitor for the manufacture of a medicament for inhibiting cancer metastasis, wherein the IL-6 inhibitor is an anti-IL-6 receptor antibody.

3. The agent for use according to claim 1 or the use according to claim 2, wherein the cancer metastasis is cancer metastasis to the liver.

4. The agent for use according to claim 1 or 3 or the use according to claim 2 or 3, wherein the anti-IL-6 receptor antibody is a chimeric, humanized, or human antibody.

5. The agent for use according to any one of claims 1, 3 and 4 or the use according to any one of claims 2 to 4, wherein the cancer metastasis is metastasis of lung cancer to the liver.

## Patentansprüche

1. Agens zur Verwendung in einem Verfahren zur Inhibierung von Krebsmetastasierung, umfassend einen Interleukin-6 (IL-6)-Inhibitor als Wirkstoff, wobei der IL-6-Inhibitor ein Anti-IL-6-Rezeptorantikörper ist.

2. Verwendung eines IL-6-Inhibitors zur Herstellung eines Medikaments zur Inhibierung von Krebsmetastasierung, wobei der IL-6-Inhibitor ein Anti-IL-6-Rezeptorantikörper ist.

3. Agens zur Verwendung nach Anspruch 1 oder Verwendung nach Anspruch 2, wobei die Krebsmetastasierung eine Krebsmetastasierung in der Leber ist.

4. Agens zur Verwendung nach Anspruch 1 oder 3 oder Verwendung nach Anspruch 2 oder 3, wobei der Anti-IL-6-Rezeptorantikörper ein chimärer, humanisierter oder humaner Antikörper ist.

5. Agens zur Verwendung nach einem der Ansprüche 1, 3 oder 4 oder Verwendung nach einem der Ansprüche 2 bis 4, wobei die Krebsmetastasierung eine Metastasierung von Lungenkrebs in der Leber ist.

## Revendications

1. Agent pour utilisation dans un procédé d'inhibition de métastase cancéreuse comprenant un inhibiteur d'interleukine 6 (IL-6) en tant que substance active, l'inhibiteur d'IL-6 étant un anticorps anti-récepteur d'IL-6.

2. Utilisation d'un inhibiteur d'IL-6 pour la fabrication d'un médicament pour inhiber la métastase cancéreuse, l'inhibiteur d'IL-6 étant un anticorps anti-récepteur d'IL-6.

3. Agent pour utilisation selon la revendication 1 ou utilisation selon la revendication 2, la métastase cancéreuse étant une métastase cancéreuse dans le foie.

4. Agent pour utilisation selon la revendication 1 ou 3 ou utilisation selon la revendication 2 ou 3, l'anticorps anti-récepteur d'IL-6 étant un anticorps chimérique, humanisé ou humain.

5. Agent pour utilisation selon l'une quelconque des revendications 1, 3 et 4 ou utilisation selon l'une quelconque des revendications 2 à 4, la métastase cancéreuse étant une métastase de cancer du poumon dans le foie.
